# EUROPEAN PATENT SPECIFICATION

(11) **EP 2 988 751 B1**
(45) Date of publication and mention of the grant of the patent: **20.02.2019**
(21) Application number: 14788546.1
(22) Date of filing: 25.04.2014
(51) Int. Cl.: A61K 9/20, A61K 9/48, A61K 47/02, A61K 47/10, A61K 47/12, A61K 47/14, A61K 47/18, A61K 47/22, A61K 47/26, A61K 47/32, A61K 31/546, A61P 31/04

(54) **ORAL CEFEPIME COMPOSITIONS AND USES THEREOF**
ORALE CEFEPIMZUSAMMENSETZUNGEN UND VERWENDUNGEN DAVON
COMPOSITIONS DE CÉFÉPIME ORALES ET UTILISATIONS CORRESPONDANTES

(30) Priority: 25.04.2013 US 201361815932 P
(43) Date of publication of application: 02.03.2016
(73) Proprietor: Seachaid Pharmaceuticals, Inc., Durham, NC 27713 (US)
(72) Inventor: RADHAKRISHNAN, Balasingam, Chapel Hill, NC 27517 (US); VAIDYA, Anuradha, Raleigh, NC 27616 (US)
(74) Representative: Carpmaels & Ransford LLP
(86) International application number: PCT/US2014/035454
(87) International publication number: WO 2014/176501

(56) References cited:
- WO-A1-2012/129493
- WO-A1-2013/059664
- US-A1- 2008 213 366
- US-A1- 2010 278 901
- US-A1- 2011 319 377
- US-B1- 6 248 363
- CHAPMAN ET AL.: 'Cefepime' AMERICAN JOURNAL OF RESPIRATORY MEDICINE., [Online] vol. 2, no. 1, 2013, pages 75 - 107, XP008181936 Retrieved from the Internet: <URL:http://link.springer.com/article/10.10 07%2FBF03256641> [retrieved on 2014-08-08]

## Description

### Background of the Invention

This invention relates to the field of treatment of bacterial infections.

To be pharmaceutically effective, an orally administered drug must be absorbed into the bloodstream through epithelial cells lining the gastrointestinal tract. Many drug candidates cannot be orally administered because of poor absorptive characteristics. Hence, the development of reagents suitable for medicinal use that improve oral bioavailability when co-administered with pharmaceuticals is a primary focus in drug development and medicinal chemistry.

A number of potent antibiotics and antibiotic prodrugs have inadequate oral bioavailability on their own. For this reason, such antibiotics are traditionally administered intravenously to treat serious infections. However, intravenous administration is an invasive procedure that is potentially detrimental to the subject (side effects include IV-line phlebitis and sepsis), inconvenient, and costly. Thus, there is a need to develop formulations that enhance the oral bioavailability of antibiotics so as to increase the number of oral antibiotics available to subjects.

CHAPMAN ET AL, "Cefepime", AMERICAN JOURNAL OF RESPIRATORY MEDICINE, vol. 2, no. 1, 2003, pages 75-107, discloses the use of cefepime in the management of pneumonia by intravenous, intramuscular and oral administration.

### Summary of the Invention

The scope of the invention is defined by the appended claims. Any embodiment described below not falling within the scope of the appended claims is described for reference purposes.

We have discovered oral dosage formulations for increasing the oral bioavailability of cefepime. The formulations include an acyl carnitine (e.g., palmitoyl carnitine), optionally in combination with an alcohol, a polysorbate surfactant, a carboxylic acid, a polyethylene glycol, a polyglycolized glyceride, alkyl saccharides, ester saccharides, a TPGS compound, or a sugar.

The invention features a pharmaceutical composition in oral dosage form comprising cefepime, or a pharmaceutically acceptable salt thereof, and an additive comprising an acyl carnitine or a salt thereof, wherein said additive is present in an amount sufficient to increase the oral bioavailability of said cefepime, or said pharmaceutically acceptable salt thereof, wherein said oral dosage form comprises a (w/w) ratio of cefepime, or a pharmaceutically acceptable salt thereof, to acyl carnitine of from 1:0.5 to 1:6 (e.g., 1:0.5 to 1:1, 1:0.5 to 1:2, 1:0.75 to 1:4, 1:1 to 1:3, 1:1 to 1:6, 1:2 to 1:5, or 1:2.5 to 1:6). The additive can further include an alcohol, a polysorbate surfactant, a carboxylic acid, a polyethylene glycol, a polyglycolized glyceride, alkyl saccharides, ester saccharides, a TPGS compound, or a sugar.

In some embodiments the unit dosage form includes from 1% to 60% (e.g., 1% to 10%, 5% to 15%, 10% to 30%, or 20% to 60%) (w/w) of an alcohol selected from propylene glycol, ethanol, or glycerol. In certain embodiments the unit dosage form includes from 1% to 10% (e.g., 1% to 3%, 2% to 5%, 3% to 7%, or 5% to 10%) (w/w) of a carboxylic acid selected from citric acid, succinic acid, tartaric acid, fumaric acid, maleic acid, malonic acid, glutaric acid, adipic acid, lactic acid, malic acid, L-glutamic acid, L-aspartic acid, gluconic acid, glucuronic acid, salicylic acid, and salts thereof. In particular embodiments the unit dosage form includes from 0.5% to 10% (e.g., 0.5% to 2%, 2% to 5%, 3% to 7%, or 5% to 10%) (w/w) of a polysorbate surfactant. In some embodiments the unit dosage form includes from 0.2% to 12% (e.g., 0.2% to 2%, 1% to 4%, 3% to 7%, or 6% to 12%) (w/w) TPGS compound. In certain embodiments the unit dosage form includes from 2% to 30% (e.g., 2% to 10%, 5% to 15%, 10% to 20%, or 15% to 30%) (w/w) polyethylene glycol. In particular embodiments the unit dosage form includes from 5% to 30% (e.g., 5% to 12%, 8% to 15%, 10% to 20%, or 15% to 30%) (w/w) alkyl saccharide or ester saccharide. In some embodiments the unit dosage form includes from 5% to 30% (e.g., 5% to 12%, 8% to 15%, 10% to 20%, or 15% to 30%) (w/w) alkyl saccharide or ester saccharide. In particular embodiments the unit dosage form includes from 5% to 60% (e.g., 5% to 20%, 10% to 35%, 20% to 40%, or 35% to 60%) (w/w) polyglycolized glyceride. In certain embodiments the unit dosage form includes from 5% to 25% (e.g., 5% to 12%, 8% to 15%, 10% to 20%, or 15% to 25%) (w/w) sugar selected from monosaccharides, disaccharides, and sugar alcohols.

In any of the above pharmaceutical compositions, the acyl carnitine can be selected from oleoyl carnitine, palmitoyl carnitine, decanoyl carnitine, dodecanoyl carnitine, myristoyl carnitine, stearoyl carnitine, and salts thereof.

The unit dosage form can be formulated as a liquid-filled gel capsule or as a sachet for reconstitution. Alternatively, the unit dosage form can include a solid or semisolid. In particular embodiments the unit dosage form is a powder (e.g., as a sachet for reconstitution).

The unit dosage form can include from 5% to 50% (e.g., 5% to 20%, 10% to 35%, 20% to 40%, or 35% to 50%) (w/w) acyl carnitine or a salt thereof.

In particular embodiments, the pharmaceutical composition is a liquid dosage form including from 1.5% to 15% (e.g., 1.5%, 3% to 5%, 4% to 8%, 5% to 12%, or 8% to 15%) (w/w) cefepime or a pharmaceutically acceptable salt thereof.

In still other embodiments, the pharmaceutical composition is a solid or semisolid dosage form including from 10% to 33% (e.g., 10% to 15%, 12% to 22%, 20% to 28%, or 25% to 33%) (w/w) cefepime or a pharmaceutically acceptable salt thereof. The solid or semisolid dosage form can include a hydrophilic matrix selected from maltose, mannitol, sucrose, crystalline cellulose, cornstarch, and methylcellulose. For example, the solid dosage form can be a tablet including a disintegrant and a hydrophilic matrix selected from maltose, mannitol, sucrose, crystalline cellulose, cornstarch, and methylcellulose.

In any of the above pharmaceutical compositions, the unit dosage form can include from 0.1% to 60% (e.g., 0.1% to 15%, 2% to 10%, 8% to 60%, or 25% to 45%) (w/w) water. The unit dosage form can be formulated as a suspension for reconstitution, or as a liquid-filled gel capsule.

The formulations can include a mixture of two or more of an alcohol, a polysorbate surfactant, a carboxylic acid, a polyethylene glycol, a polyglycolized glyceride, alkyl saccharides, ester saccharides, a TPGS compound, or a sugar in combination with the acyl carnitine to enhance the oral bioavailability of the cefepime. For example, the formulation can include a mixture of a TPGS compound, a polyglycolized glyceride, and an acyl carnitine.

The formulations include an acyl carnitine (e.g., palmitoyl carnitine), optionally in combination with an alcohol, a polysorbate surfactant, a carboxylic acid, a polyethylene glycol, a polyglycolized glyceride, alkyl saccharides, ester saccharides, a TPGS compound, or a sugar.

In certain embodiments of the pharmaceutical compositions of the invention, the cefepime, or a pharmaceutically acceptable salt thereof, and the additive are adsorbed onto a solid matrix (e.g., a porous silicate including alkali-metal silicates, alkaline earth metal silicates, or aluminum silicates, or including aluminum silicate, magnesium aluminum silicate, sodium silicate, potassium silicate, magnesium silicate, or calcium silicate), or any other solid matrix described herein. In particular embodiments the porous silicate is Neusilin. In particular embodiments, the pharmaceutical composition further comprises from 0.2% to 5% (w/w) chloride salt (e.g., an inorganic chloride salt, such as lithium chloride, sodium chloride, potassium chloride, magnesium chloride, or calcium chloride; or an organic chloride salt, such amine hydrochloride salts).

In particular embodiments, the reconstitutable pharmaceutical compositions of the invention (e.g., dry blends and liquid concentrates) have a shelf-life of at least 2 weeks, 1 month, 2 months, 3 months, or 6 months, as determined by the amount of time it takes 3% of the cefepime in the formulation to degrade during storage at room temperature in the dark. In other embodiments, the amount of cefepime in the reconstitutable pharmaceutical compositions of the invention (e.g., dry blends and liquid concentrates) is reduced by less than 3%, 2%, or 1% when stored at room temperature in the dark for 2 weeks, 1 month, 2 months, 3 months, or 6 months. For example, the amount of cefepime in the reconstitutable pharmaceutical composition can be reduced by less than 2% or 1% when stored at room temperature in the dark for 2 months. In particular embodiments, the stable reconstitutable pharmaceutical compositions include from 50% (w/w) to 75% (w/w) acyl carnitine and from 15% (w/w) to 25% (w/w) cefepime, or a salt thereof. Optionally, the stable reconstitutable pharmaceutical compositions include from 10% (w/w) to 20% (w/w) carboxylic acid or a salt thereof, such as citric acid, succinic acid, tartaric acid, fumaric acid, maleic acid, malonic acid, glutaric acid, adipic acid, lactic acid, malic acid, L-glutamic acid, L-aspartic acid, gluconic acid, glucuronic acid, salicylic acid, or salts thereof.

For example, the pharmaceutical composition can be a powder (e.g., in sachet for reconstitution).

In one particular embodiment of the above pharmaceutical compositions, the unit dosage form contains no polyglycolized glyceride. In another particular embodiment of the above pharmaceutical compositions, the unit dosage form contains no fatty acid monoester or diester of a polyether (e.g., polyethylene glycol or polypropylene glycol esterified with one or two fatty acids). In still another particular embodiment of the above pharmaceutical compositions, the unit dosage form contains no alkyl saccharides. In one particular embodiment of the above pharmaceutical compositions, the unit dosage form contains no ester saccharides. Optionally, the unit dosage form contains less than 5%, 4%, 3%, 2%, or 1% (w/w) of polyglycolized glycerides, fatty acid monoesters or diesters of a polyether, alkyl saccharides, and/or ester saccharides.

The invention also features the above pharmaceutical composition for use in a method of treating a bacterial infection in a subject, said method comprising orally administering to said subject the pharmaceutical composition, wherein said composition is administered in an amount effective to treat said infection.

In some embodiments, the bacterial infection is pneumonia, upper and lower respiratory tract infection, uncomplicated skin and skin structure infection, complicated skin and skin structure infection, soft tissue infection, bone and joint infection, diabetic foot infections, hospital-acquired lung infection, acute bacterial otitis media, bacterial pneumonia, complicated infection, noncomplicated infection, pyelonephritis, uncomplicated intra-abdominal infection, complicated intra-abdominal infection, deep-seated abcess, bacterial sepsis, central nervous system infection, bacteremia, wound infection, peritonitis, meningitis, infections after burn, uncomplicated and complicated urinary tract infection, gastro-intestinal tract infection, pelvic inflammatory disease, endocarditis, febrile neutropenia, and intravascular infection. In preferred embodiments, the infection is pneumonia, febrile neutropenia, uncomplicated or complicated urinary tract infection, uncomplicated skin and skin structure infection, or complicated intra-abdominal infection.

In particular embodiments, the bacterial infection is a venereal disease. For example, the bacterial infection can be chancroid, Chlamydia, gonorrhea, granuloma inguinale, or syphilis.

In other embodiments, the bacterial infection is caused by *Streptococcus* spp (including *Streptococcus pneumoniae* and *Streptococcus pyogenes*), *Pseudomonas aeruginosa, Klebsiella* spp (including *Klebsiella pneumoniae*), *Enterobacter* spp, *Escherichia coli, Proteus* spp (including *Proteus mirabilis*), *Staphylococcus* spp (including *Staphylococcus aureus*), *Acinetobacter* spp, *Bacteroides* spp (including *Bacteroides fragilis*), *Citrobacter* spp, *Providencia* spp, *Serratia marcescens, Haemophilus influenzae, Morganella morganii*, *Hafnia alvei, Moraxella catarrhalis, Haemophilus ducreyi, Chlamydia trachomatis, Neisseria gonorrhoeae, Klebsiella granulomatis, Treponema pallidum,* viridans group streptococci, or Mycobacterium spp.

In an embodiment of the pharmaceutical composition of the invention for use in a method of treating a bacterial infection in a subject, the subject receives intravenous antibiotics (e.g., an intravenous cephalosporin, or an intravenous carbapenem treatment) prior to the oral administration of the pharmaceutical composition of the invention. Thus, the methods and compositions of the invention can be used to compliment intravenous antibiotic dosing regimens, allowing the subject to transition to an oral dosing regimen as an out-patient treatment.

Described also are kits, including: a) any composition of the invention; and b) instructions for administering the composition to a subject diagnosed with a bacterial infection.

By "acyl carnitine" is meant a chemical moiety with the formula: 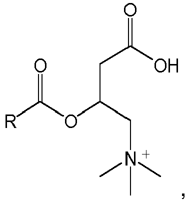 and salts thereof, wherein R is a partially-saturated straight chain or branched hydrocarbon group having between 8 and 26 carbon atoms. Acyl carnitines are derived from carnitine (D or L form, or a mixture thereof) and a fatty acid. The acyl carnitine can be an ester of a fatty acid having 16 carbon atoms and 0, 1 or 2 double bonds (C16:0; C16:1 and C16:2), those with 18 carbon atoms and 1, 2 or 3 double bonds (C18:1; C18:2; and C18:3), those with 20 carbon atoms and 1, 2 or 4 double bonds (C20:1; C20:2; and C20:4), or those with 22 carbon atoms and 4, 5 or 6 double bonds (C22:4; C22:5 and C22:6). Acyl carnitines include, without limitation, 4, 7, 10, 13, 16, 19 docosahexanoyl carnitine, oleoyl carnitine, palmitoyl carnitine, decanoyl carnitine, dodecanoyl carnitine, myristoyl carnitine, and stearoyl carnitine. In a preferred embodiment, the pharmaceutical composition of the invention includes palmitoyl carnitine or a pharmaceutically acceptable salt thereof.

As used herein, the term "administration" or "administering" refers to peroral administration of a drug to a subject.

By "additive" is meant those components of a pharmaceutical composition containing cefepime, or a pharmaceutically acceptable salt thereof, in an oral dosage form which increase the oral bioavailability of the drug when orally administered simultaneously with the drug. Additives of the invention include an acyl carnitine and, optionally, an alcohol, a polysorbate surfactant, a carboxylic acid, a polyethylene glycol, a polyglycolized glyceride, alkyl saccharides, ester saccharides, a TPGS compound, or a sugar.

By "an amount sufficient" is meant the amount of an additive required to increase the oral bioavailability of a drug.

By "bacterial infection" is meant the invasion of a host by pathogenic bacteria. For example, the infection may include the excessive growth of bacteria that are normally present in or on the body of a human or growth of bacteria that are not normally present in or on a human. More generally, a bacterial infection can be any situation in which the presence of a bacterial population(s) is damaging to a host body. Thus, a human is "suffering" from a bacterial infection when an excessive amount of a bacterial population is present in or on the person's body, or when the presence of a bacterial population(s) is damaging the cells or other tissue of the person.

As used herein, the term "drug particle" refers to microparticles or nanoparticles containing cefepime, or a salt thereof. The drug particles can be included in the unit dosage forms of the invention. For example, the unit dosage form can be a capsule containing nanoparticulates containing cefepime, or a salt thereof.

By "effective" amount is meant the amount of drug required to treat or prevent an infection or a disease associated with an infection. The effective amount of drug used to practice the invention for therapeutic or prophylactic treatment of conditions caused by or contributed to by a microbial infection varies depending upon the manner of administration, the age, body weight, and general health of the subject. Ultimately, the attending physician will decide the appropriate amount and dosage regimen. Such amount is referred to as an "effective" amount.

As used herein, the terms "effective particle size" and "particle size" are used interchangeably and refer to a mixture of particles having a distribution in which 50% of the particles are below and 50% of the particles are above a defined measurement. The "effective particle size" refers to the volume-weighted median diameter as measured by a laser/light scattering method or equivalent, wherein 50% of the particles, by volume, have a smaller diameter, while 50% by volume have a larger diameter. The effective particle size can be measured by conventional particle size measuring techniques well known to those skilled in the art. Such techniques include, for example, sedimentation field flow fractionation, photon correlation spectroscopy, light scattering (e.g., with a Microtrac UPA 150), laser diffraction, and disc centrifugation.

By "hard capsule" is meant a capsule that includes a membrane that forms a two-part, capsule-shaped, container capable of carrying a solid, semi-solid, or liquid payload of drug, additive(s), and, optionally, excipients.

By "liquid dosage form" is meant a solution or suspension from which a dose is measured out (i.e., a teaspoon, tablespoon, or a number of cubic centimeters) for oral administration to a subject.

By "microgranule" is meant a heterogenous solid particle having a diameter between 50 and 1000 microns.

As used herein, "oral bioavailability" refers to the fraction of drug absorbed following oral administration to a subject as measured by the blood circulating concentration in comparison to the blood circulating concentration observed for the 100% bioavailability observed with intravenously or intraarterially administered drug.

By "polyglycolized glyceride" is meant a polyethylene glycol glyceride monoester, a polyethylene glycol glyceride diester, a polyethylene glycol glyceride triester, or a mixture thereof containing a variable amount of free polyethylene glycol, such as a polyethylene glycol-oil transesterification product. The polyglycolized glyceride can include either monodisperse (i.e., single molecular weight) or polydisperse polyethylene glycol moieties of a predetermined size or size range (e.g., PEG2 to PEG 40). Polyethylene glycol glycerides include, for example: PEG glyceryl caprate, PEG glyceryl caprylate, PEG-20 glyceryl laurate (Tagat® L, Goldschmidt), PEG-30 glyceryl laurate (Tagat® L2, Goldschmidt), PEG-15 glyceryl laurate (Glycerox L series, Croda), PEG-40 glyceryl laurate (Glycerox L series, Croda), PEG-20 glyceryl stearate (Capmul® EMG, ABITEC), and Aldo® MS-20 KFG, Lonza), PEG-20 glyceryl oleate (Tagat® O, Goldschmidt), and PEG-30 glyceryl oleate (Tagat® O2, Goldschmidt). Caprylocapryl PEG glycerides include, for example, caprylic/capric PEG-8 glyceride (Labrasol®, Gattefosse), caprylic/capric PEG-4 glyceride (Labrafac® Hydro, Gattefosse), and caprylic/capric PEG-6 glyceride (SOFTIGEN®767, Huls). Oleoyl PEG glycerides include, for example, oleoyl PEG-6 glyceride, (Labrafil M1944 CS, Gattefosee). Lauroyl PEG glycerides include, for example, lauroyl PEG-32 glyceride (Gelucire® ELUCIRE 44/14, Gattefosse). Stearoyl PEG glycerides include, for example stearoyl PEG-32 glyceride (Gelucire 50/13, Gelucire 53/10, Gattefosse). PEG castor oils include PEG-3 castor oil (Nikkol CO-3, Nikko), PEG-5, 9, and 16 castor oil (ACCONON CA series, ABITEC), PEG-20 castor oil, (Emalex C-20, Nihon Emulsion), PEG-23 castor oil (Emulgante EL23), PEG-30 castor oil (Incrocas 30, Croda), PEG-35 castor oil (Incrocas-35, Croda), PEG-38 castor oil (Emulgante EL 65, Condea), PEG-40 castor oil (Emalex C-40, Nihon Emulsion), PEG-50 castor oil (Emalex C-50, Nihon Emulsion), PEG-56 castor oil (Eumulgin® PRT 56, Pulcra SA), PEG-60 castor oil (Nikkol CO-60TX, Nikko), PEG-100 castor oil, PEG-200 castor oil (Eumulgin® PRT 200, Pulcra SA), PEG-5 hydrogenated castor oil (Nikkol HCO-5, Nikko), PEG-7 hydrogenated castor oil (Cremophor WO7, BASF), PEG-10 hydrogenated castor oil (Nikkol HCO-10, Nikko), PEG-20 hydrogenated castor oil (Nikkol HCO-20, Nikko), PEG-25 hydrogenated castor oil (Simulsol® 1292, Seppic), PEG-30 hydrogenated castor oil (Nikkol HCO-30, Nikko), PEG-40 hydrogenated castor oil (Cremophor RH 40, BASF), PEG-45 hydrogenated castor oil (Cerex ELS 450, Auschem Spa), PEG-50 hydrogenated castor oil (Emalex HC-50, Nihon Emulsion), PEG-60 hydrogenated castor oil (Nikkol HCO-60, Nikko), PEG-80 hydrogenated castor oil (Nikkol HCO-80, Nikko), and PEG-100 hydrogenated castor oil (Nikkol HCO-100, Nikko). Additional polyethylene glycol-oil transesterification products include, for example, stearoyl PEG glyceride (Gelucire® 50/13, Gattefosse). The polyglycolized glycerides useful in the formulations of the invention can include polyethylene glycol glyceride monoesters, diesters, and/or triesters of acetic, propionic, butyric, valeric, hexanoic, heptanoic, caprylic, nonanoic, capric, lauric, myristic, palmitic, heptadecanoic, stearic, arachidic, behenic, lignoceric, α-linolenic, stearidonic, eicosapentaenoic, docosahexaenoic, linoleic, γ-linolenic, dihomo-γ-linolenic, arachidonic, oleic, elaidic, eicosenoic, erucic, or nervonic acid, or mixtures thereof. The polyglycol moiety in a polyglycolized glyceride can be polydisperse; that is, they can have a variety of molecular weights.

By "TPGS compound" is meant a compound or mixture of compounds containing one or more vitamin E moieties (e.g., a tocopherol, tocomonoenol, tocodienol, or tocotrienol) bonded to (e.g., by an ester, amide, or thioester bond) to one or more polyethylene glycol (PEG) moieties via a linker (e.g., a dicarboxylic or tricarboxylic acid). The vitamin E moiety can be any naturally occurring or synthetic form of vitamin E, including α-, β-, γ-, and δ- isoforms and all stereoisomers of tocopherol, tocomonoenol, tocodienol, and tocotrienol. Linkers include, for example, dicarboxylic acids (e.g., succinic acid, sebacic acid, dodecanedioic acid, suberic acid, or azelaic acid, citraconic acid, methylcitraconic acid, itaconic acid, maleic acid, glutaric acid, glutaconic acid, fumaric acids and phthalic acids). Exemplary tocopherol polyethylene glycol diesters are TPGS, tocopherol sebacate polyethylene glycol, tocopherol dodecanodioate polyethylene glycol, tocopherol suberate polyethylene glycol, tocopherol azelaate polyethylene glycol, tocopherol citraconate polyethylene glycol, tocopherol methylcitraconate polyethylene glycol, tocopherol itaconate polyethylene glycol, tocopherol maleate polyethylene glycol, tocopherol glutarate polyethylene glycol, tocopherol glutaconate polyethylene glycol, and tocopherol phthalate polyethylene glycol. Each of the PEG moieties of the TPGS compound can be any polyethylene glycol or any PEG derivative, and can have a molecular weight of 200-6000 kDa (e.g., 400-4000 kDa, 500-2000 kDa, 750-1500 kDa, 800-1200 kDa, 900-1100 kDa, or about 1000 kDa). The PEG moieties can be polydisperse; that is, they can have a variety of molecular weights. PEG derivatives include, for example, methylated PEG, propylene glycol, PEG-NHS, PEG-aldehyde, PEG-SH, PEG-NH₂, PEG-CO₂H, PEG-OMe and other ethers, branched PEGs, and PEG copolymers (e.g., PEG-b-PPG-b-PEG-1100, PEG-PPG-PEG-1900, PPG-PEG-MBE-1700, and PPG-PEG-PPG-2000). Any known source of TPGS compound can be used in the present invention. An exemplary TPGS compound is tocopheryl PEG-1000 succinate (TPGS-1000), which has a PEG moiety having a molecular weight of 1000 kDa. A food grade TPGS-1000 is available, for example, under the trade name Eastman Vitamin E TPGS® (Eastman Chemical Company, Kingsport, Tennessee). This TPGS is water-soluble form of natural-source vitamin E, which is prepared by esterification of crystalline D-α-tocopheryl acid succinate with polyethylene glycol 1000 (PEG 1000), and contains between 260 and 300 mg/g total tocopherol. Another exemplary TPGS compound is Water Soluble Natural Vitamin E (ZMC-USA, The Woodlands, Texas). Methods of preparing TPGS are described in U.S. Patent Nos. 2,680,749 and 3,102,078 and in U.S. Publication Nos. 2007/0184117 and 2007/0141203. TPGS compounds also include TPGS analogs that differ in chemical composition from TPGS by the substitution, addition, or removal of one or more atoms, methylene (CH₂)ₙ units, or functional groups. TPGS analogs also include chromanol derivatives (e.g., 6-chromanol PEG-1000 succinate and 6-chromanol PEG-400 succinate), steroid derivatives (e.g., cholesteryl PEG-1000 succinate, cholic acid PEG-1000, dihydro cholic acid PEG-1000, litho-cholic acid PEG-1000, ursodeoxycholic acid PEG-1000, chenodeoxycholic acid PEG-1000), and others (e.g., indomethacin PEG-1000, chromone-2-carboxylic acid PEG-1000, chromone-2-carboxylic acid PEG-1100-OMe, chromone-2-carboxylic acid PEG-1500, chromone-2-carboxylic acid PEG-2000, naproxen PEG-1000, probenecid PEG-1000, 7-carboxymethoxy-4-methyl-coumarin PEG-1000, 5-(4-chlorophenyl)-2-furoic acid PEG-1000, probenecid tocopheryl PEG-1000 succinate, lithocholic acid PEG-1000, and chromone-3-carboxylic acid PEG-1000, 7-hydroxy-coumarinyl-4-acetic acid PEG-1000).

As used herein, the term "salt" refers to any pharmaceutically acceptable salt, such as a non-toxic acid addition salt, metal salt, or metal complex, commonly used in the pharmaceutical industry. Examples of acid addition salts include organic acids, such as acetic, lactic, pamoic, maleic, citric, malic, ascorbic, succinic, benzoic, palmitic, suberic, salicylic, tartaric, methanesulfonic, toluenesulfonic, or trifluoroacetic acids, and inorganic acids, such as hydrochloric acid, hydrobromic acid, sulfuric acid, and phosphoric acid. Representative alkali or alkaline earth metal salts include sodium, lithium, potassium, calcium, and magnesium, among others.

By "soft capsule" is meant a capsule molded into a single container carrying a liquid payload of drug, additive(s), and, optionally, excipients.

By "subject" is meant an animal, e.g., a human.

As used herein, the term "treating" refers to administering a pharmaceutical composition for prophylactic and/or therapeutic purposes. To "prevent disease" refers to prophylactic treatment of a subject who is not yet ill, but who is susceptible to, or otherwise at risk of, a particular disease. To "treat disease" or use for "therapeutic treatment" refers to administering treatment to a subject already suffering from a disease to improve or stabilize the subject's condition. Thus, in the claims and embodiments, treating is the administration to a subject either for therapeutic or prophylactic purposes.

The term "unit dosage form" refers to physically discrete units suitable as unitary dosages, such as a tablet, caplet, hard capsule, soft capsule, or sachet, each unit containing a predetermined quantity of drug.

Other features and advantages of the invention will be apparent from the following detailed description, the drawings, and the claims.

### Brief Description of the Drawings

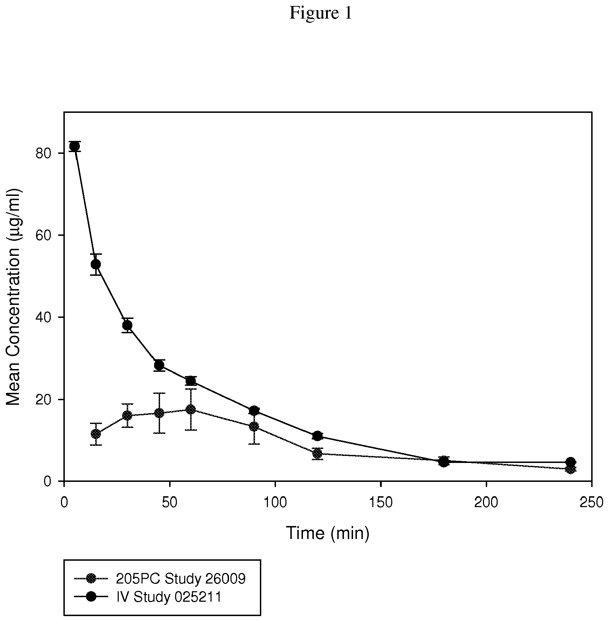

Figure 1 is a graph depicting a PK curve observed for formulation 4 administered orally, along with the curve for cefepime administered intravenously (10 mg/kg). The area under the curve for the two measurements was used to determine the oral bioavailability of cefepime formulated as described for formulation 4 in Table 1 (see Example 1).

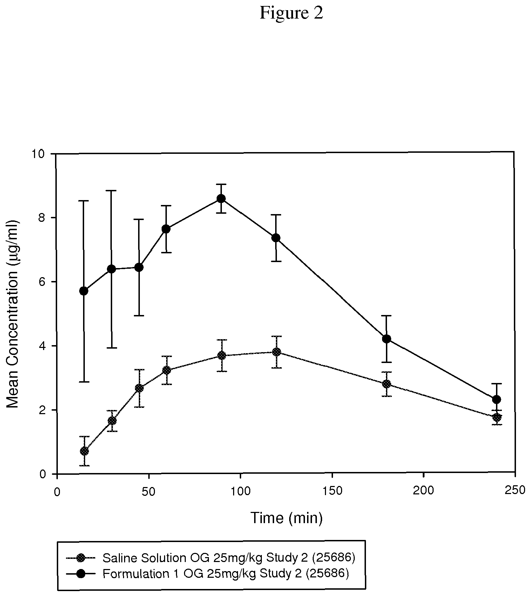

Figure 2 is a graph depicting a PK curve observed for formulation 1 administered orally, along with the curve for cefepime formulated in saline. The area under the curve for the two measurements was used to determine the oral bioavailability of cefepime formulated as described for formulation 1 in Table 1 (see Example 1).

### Detailed Description

The scope of the present invention is defined by the appended claims. Any embodiment described below not falling within the scope of the appended claims is described for reference purposes.

The invention features pharmaceutical compositions and their use in methods of treating a bacterial infection in a subject, the compositions including cefepime, or a pharmaceutically acceptable salt thereof, and an additive including an acyl carnitine, optionally in combination with an alcohol, a polysorbate surfactant, a carboxylic acid, a polyethylene glycol, a polyglycolized glyceride, alkyl saccharides, ester saccharides, a TPGS compound, or a sugar. The additive is present in an amount sufficient to increase the oral bioavailability of the cefepime. The (w/w) ratio of cefepime, or a pharmaceutically acceptable salt thereof, to acyl carnitine is of from 1:0.5 to 1:6. Cefepime is a β-lactam antibiotic with broad-spectrum activity against Gram-positive and Gram-negative bacteria. 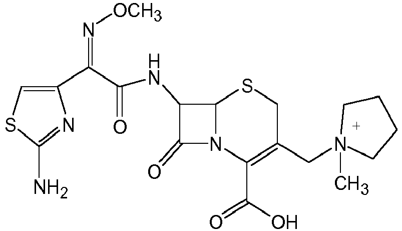 cefepime

The formulations of the invention can be used to enhance the oral bioavailability of cefepime.

### Formulation

The present invention features formulations having additives including an acyl carnitine. These additives increase the oral bioavailability of cefepime and pharmaceutically acceptable salts thereof. The formulations include an acyl carnitine, optionally in combination with an alcohol, a polysorbate surfactant, a carboxylic acid, or a salt thereof, a polyethylene
glycol, a polyglycolized glyceride, alkyl saccharides, ester saccharides, a TPGS compound, or a sugar. The (w/w) ratio of cefepime, or a pharmaceutically acceptable salt thereof, to acyl carnitine is of from 1:0.5 to 1:6.

### TPGS compounds

Tocopheryl polyethylene glycol succinate (tocopheryl PEG-1000 succinate) and related TPGS compounds can be used in the oral dosage forms of the invention. Tocopheryl PEG-1000 succinate has the following structure: 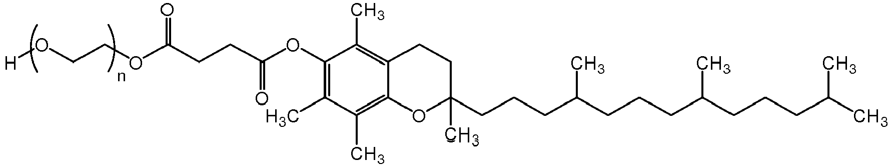 where n is an integer.

Related TPGS compounds include additives formed using different diacid linkers, different length polyethylene glycol tails, and different isoforms (e.g. α-, β-, γ-, or δ-) of tocopherol, tocomonoenol, tocodienol, and tocotrienol. These include α-tocopherol, α-tocomonoenol, α-tocodienol, α-tocotrienol, β-tocopherol, β-tocomonoenol, β-tocodienol, β-tocotrienol, γ-tocopherol, γ-tocomonoenol, γ-tocodienol, γ-tocotrienol, δ-tocopherol, δ-tocomonoenol, δ-tocodienol, δ-tocotrienol, and any stereoisomer thereof. Suitable vitamin E compounds of the present invention also include desmethyl-tocopherol, desmethyl-tocomonoenol, desmethyl-tocodienol, desmethyl-tocotrienol, and any stereoisomer thereof. Furthermore, when a compound disclosed herein contains one or more chiral atoms where stereochemistry is unspecified, it will be understood that each stereoisomer of the compound is invidually disclosed as if the structure of each stereoisomer were explicitly drawn. In certain embodiments of the invention, the vitamin E compound may be a naturally-occuring d-stereoisomer of vitamin E.

The vitamin E moieties of the present invention may be naturally occurring or synthetic. Certain embodiments of the invention include a naturally occurring vitamin E compound such as an extract from a food source. For example, α-tocopherol, α-tocotrienol, β-tocopherol, β-tocotrienol, γ-tocopherol, γ-tocotrienol, δ-tocopherol, and δ-tocotrienol are available naturally from fortified cereals, green vegetables, nuts, seeds, and vegetable oils. Methods of extracting vitamin E from natural sources have been described, for example, in U.S. Patent Nos. 6,743,450; 6,838,104; 7,161,055; and 7,544,822.

The TGPS compound can include synthetic vitamin E moieties. An exemplary method for making α-tocopherol is the reaction of trimethylhydroquinone (TMHQ) with iso-phytol (3,7,11,15-tetramethylhexadec-1-en-3-ol) in a condensation reaction with a catalyst. It will be apparent to one skilled in the art that other tocopherol, tocomonoenol, tocodienol, and tocotrienol isoforms and their derivates can also be prepared using a similar strategy starting from appropriate precursors. For example, the starting compounds may be TMHQ and 3,7,11,15-tetramethylhexadec-2-en-1-ol. An additional method of making vitamin E with isophytol under relatively mild conditions has been described by Wehrli et al., J. Org. Chem. 36:2910 (1971). Methods for synthesizing unsaturated side chains of vitamin E are described in U.S. Patent No. 4,168,271. Additional methods of synthesizing vitamin E side chains have been reviewed by Stalla-Bourdillon, Ind. Chim. Belg. 35, 13 (1970). Additional methods of synthesizing tocopherols are described in U.S. Patent Nos. 5,523,420, and 6,005,122. Additional methods of synthesizing tocotrienols are described in U.S. Patent No. 7,038,067.

The TGPS compounds can include different linkers, for example, dicarboxylic acids (e.g., succinic acid, sebacic acid, dodecanedioic acid, suberic acid, or azelaic acid, citraconic acid, methylcitraconic acid, itaconic acid, maleic acid, glutaric acid, glutaconic acid, fumaric acids and phthalic acids). Exemplary tocopherol polyethylene glycol diesters are TPGS, tocopherol sebacate polyethylene glycol, tocopherol dodecanodioate polyethylene glycol, tocopherol suberate polyethylene glycol, tocopherol azelaate polyethylene glycol, tocopherol citraconate polyethylene glycol, tocopherol methylcitraconate polyethylene glycol, tocopherol itaconate polyethylene glycol, tocopherol maleate polyethylene glycol, tocopherol glutarate polyethylene glycol, tocopherol glutaconate polyethylene glycol, and tocopherol phthalate polyethylene glycol.

The PEG moiety of the TPGS compound can be any polyethylene glycol or derivative thereof, and can have a molecular weight of 200-6000 kDa (e.g., 400-4000 kDa, 500-2000 kDa, 750-1500 kDa, 800-1200 kDa, 900-1100 kDa, or about 1000 kDa). PEG derivatives include, for example, methylated PEG, polypropylene glycol (PPG), PEG-NHS, PEG-aldehyde, PEG-SH, PEG-NH₂, PEG-CO₂H, PEG-OMe and other ethers, branched PEGs, and PEG copolymers (e.g., PEG-b-PPG-b-PEG-1100, PEG-PPG-PEG-1900, PPG-PEG-MBE-1700, and PPG-PEG-PPG-2000).

Any known source of TPGS compound can be used in the present invention. TPGS typically has an HLB value of between 16 or about 16 and 18 or about 18. An exemplary TPGS compound is tocopheryl PEG-1000 succinate (also referred to herein as "TPGS 1000"), which has a PEG moiety having a molecular weight of 1000 kDa. A food grade TPGS 1000 is available, for example, under the trade name Eastman Vitamin E TPGS® (Eastman Chemical Company, Kingsport, Tennessee). This TPGS is water-soluble form of natural-source vitamin E, which is prepared by esterification of crystalline D-α-tocopheryl acid succinate with polyethylene glycol 1000 (PEG 1000), and contains between 260 and 300 mg/g total tocopherol. Another exemplary TPGS compound is Water Soluble Natural Vitamin E (ZMC-USA, The Woodlands, Texas). Methods of preparing TPGS are described in U.S. Patent Nos. 2,680,749 and 3,102,078 and in U.S. Publication Nos. 2007/0184117 and 2007/0141203.

TPGS analogs also include chromanol derivatives (e.g., 6-chromanol PEG-1000 succinate and 6-chromanol PEG-400 succinate), steroid derivatives (e.g., cholesteryl PEG-1000 succinate, cholic acid PEG-1000, dihydro cholic acid PEG-1000, litho-cholic acid PEG-1000, ursodeoxycholic acid PEG-1000, chenodeoxycholic acid PEG-1000), and others (e.g., indomethacin PEG-1000, chromone-2-carboxylic acid PEG-1000, chromone-2-carboxylic acid PEG-1 100-OMe, chromone-2-carboxylic acid PEG-1500, chromone-2-carboxylic acid PEG-2000, naproxen PEG-1000, probenecid PEG-1000, 7-carboxymethoxy-4-methyl-coumarin PEG-1000, 5-(4-chlorophenyl)-2-furoic acid PEG-1000, probenecid tocopheryl PEG-1000 succinate, lithocholic acid PEG-1000, and chromone-3-carboxylic acid PEG-1000, 7-hydroxy-coumarinyl-4-acetic acid PEG-1000).

### Alkyl saccharides

Alkyl saccharides can be used in the oral dosage forms of the invention. Alkyl saccharides are sugar ethers of a hydrophobic alkyl group (e.g., typically from 9 to 24 carbon atoms in length). Alkyl saccharides include alkyl glycosides and alkyl glucosides. In particular embodiments, the cefepime is formulated with a C₈₋₁₄ alkyl ether of a sugar. Alkyl glycosides that can be used in the oral dosage forms of the invention include, without limitation, C₈₋₁₄ alkyl (e.g., octyl-, nonyl-, decyl-, undecyl-, dodecyl-, tridecyl-, or tetradecyl-) ethers of α or β -D-maltoside, -glucoside or -sucroside, alkyl thiomaltosides, such as heptyl, octyl, dodecyl-, tridecyl-, and tetradecyl-β-D-thiomaltoside; alkyl thioglucosides, such as heptyl- or octyl 1-thio α- or β-D-glucopyranoside; alkyl thiosucroses; and alkyl maltotriosides. For example, the echinocandin class compound can be formulated with octyl maltoside, dodecyl maltoside, tridecyl maltoside, or tetradecyl maltoside. Alkyl glucosides that can be used in the oral dosage forms of the invention include, without limitation, C₈₋₁₄ alkyl (e.g., octyl-, nonyl-, decyl-, undecyl-, dodecyl-, tridecyl-, or tetradecyl-) ethers of glucoside, such as dodecyl glucoside or decyl glucoside.

### Ester saccharides

Ester saccharides can be used in the oral dosage forms of the invention. Ester saccharides are sugar esters of a hydrophobic alkyl group (e.g., typically from 8 to 24 carbon atoms in length). Ester saccharides include ester glycosides and ester glucosides. In particular embodiments, the cefepime is formulated with a C₈₋₁₄ alkyl ester of a sugar. Ester glycosides that can be used in the oral dosage forms of the invention include, without limitation, C₈₋₁₄ alkyl (e.g., octyl-, nonyl-, decyl-, undecyl-, dodecyl-, tridecyl-, or tetradecyl-) esters of α or β -D-maltoside, -glucoside or -sucroside. For example, the echinocandin class compound can be formulated with sucrose mono-dodecanoate, sucrose mono-tridecanoate, or sucrose mono-tetradecanoate. Ester glucosides that can be used in the oral dosage forms of the invention include, without limitation, C₈₋₁₄ alkyl (e.g., octyl-, nonyl-, decyl-, undecyl-, dodecyl-, tridecyl-, or tetradecyl-) esters of glucoside, such as glucose dodecanoate or glucose decanoate.

### Carboxylic Acids and Salts Thereof

Carboxylic acids can be used in the oral dosage forms of the invention. Preferred carboxylic acids include citric acid, succinic acid, tartaric acid, fumaric acid, maleic acid, malonic acid, glutaric acid, adipic acid, lactic acid, malic acid, L-glutamic acid, L-aspartic acid, gluconic acid, glucuronic acid, salicylic acid, and pharmaceutically acceptable salts thereof.

### Polyethylene Glycols

Polyethylene glycols can be used in the oral dosage forms of the invention. Preferred polyethylene glycols include PEG2 to PEG 5000 (e.g., PEG 200, PEG 400, PEG 800, PEG 1,200, and mixtures thereof).

### Polysorbate surfactants

Polysorbate surfactants can be used in the oral dosage forms of the invention. Polysorbate surfactants are oily liquids derived from pegylated sorbitan esterified with fatty acids. Common brand names for Polysorbates include Alkest, Canarcel and Tween. Polysorbate surfactants include, without limitation, polyoxyethylene 20 sorbitan monolaurate (TWEEN 20), polyoxyethylene (4) sorbitan monolaurate (TWEEN 21), polyoxyethylene 20 sorbitan monopalmitate (TWEEN 40), polyoxyethylene 20 sorbitan monostearate (TWEEN 60); and polyoxyethylene 20 sorbitan monooleate (TWEEN 80).

### Taste-masked formulations

Taste-masked formulations can be prepared by adsorbing the additive and cefepime, or a pharmaceutically acceptable salt thereof, onto a matrix (e.g., an organic matrix or inorganic matrix) to form a solid complex containing the liquid additive and cefepime, or a pharmaceutically acceptable salt thereof. Exemplary organic matrices that can be used in the tast-masked formulations of the invention include, without limitation, cellulose acetate, amorphous cellulose, starch, polyurethanes, polyvinyl alcohol, polyacrylates, mannitol, Avicel PH101, and Avicel PH102. Exemplary inorganic matrices that can be used in the tast-masked formulations of the invention include, without limitation, silica (e.g., Aerosil, Aeroperl, amorphous silica, colloidal silica), silicates (e.g., Neusilin, hectrorite), carbonates (e.g., magnesium carbonate), and metal oxides (e.g., magnesium oxide).

For example, taste-masked formulations can be prepared by adsorbing the additive and cefepime, or a pharmaceutically acceptable salt thereof, onto a porous silicate (see PCT Publication No. WO 00/38655). The porous silicate can be a swelling clay of the smectite type (e.g., bentonite, veegum, laponite), hydrous aluminium silicates or alkaline earth silicates (e.g., Neusilin, hectrorite, among others), or a porous silica gel (e.g., Syloid, Porasil, Lichrosorp). In a typical taste-masked formulation the additive and cefepime, or a pharmaceutically acceptable salt thereof, are adsorbed onto silicate selected from sodium silicate, potassium silicate, magnesium silicate, calcium silicate (including synthetic calcium silicate such as, e.g., Hubersorp), zinc silicate, aluminum silicate, sodium aluminosilicate such as, for example, Zeolex, magnesium aluminum silicate, magnesium aluminum metasilicate, aluminum metasilicate, Neusilin UFL2 (type 1-A), Neusilin (SG2), Neusilin (F1), and Neusilin (US2), or mixtures thereof.

The taste-masked formulation can be designed to form a powder that is reconstitutable in water. The incorporation of the additive and cefepime, or a pharmaceutically acceptable salt thereof, into the matrix minimizes contact with the taste buds of the subject and allows the taste of the formulation to be controlled with one or more additional flavorings agents (e.g., lemon, menthol, artificial fruity flavors etc.) and sweeteners (e.g., sugars, sugar alcohols, aspartame, etc.).

The taste-masked formulation can be designed to form a powder that is reconstitutable in water. The incorporation of carefully selected additives allows the taste of the formulation to be controlled with one or more additional flavorings agents (e.g., lemon, menthol, artificial fruity flavors etc.) and sweeteners (e.g., sugars, sugar alcohols, aspartame, etc.).

### Particulate formulations

The pharmaceutical formulations described herein can include drug particles (e.g., particles of cefepime, or salts thereof) having an effective particle size of from about 1 micron to about 50 microns. In an alternative approach, the pharmaceutical formulations described herein can include drug particles having an effective particle size of less than about 1 micron (i.e., nanoparticulate formulations).

The drug particles can be made by using any method known in the art for achieving the desired particle sizes. Useful methods include, for example, milling, homogenization, supercritical fluid fracture, or precipitation techniques. Exemplary methods are described in U.S. Patent Nos. 4,540,602; 5,145,684; 5,518,187; 5,718,388; 5,862,999; 5,665,331; 5,662,883; 5,560,932; 5,543,133; 5,534,270; and 5,510,118; 5,470,583.

In one approach, the drug, or a salt thereof, is milled in order to obtain micron or submicron particles. The milling process can be a dry process, e.g., a dry roller milling process, or a wet process, i.e., wet-grinding. A wet-grinding process is described in U.S. Patent Nos. 4,540,602; 5,145,684; 6,976,647; and EP Patent Publication No. EP498482. Thus, the wet grinding process can be practiced in conjunction with a liquid dispersion medium and dispersing or wetting agents such as described in these publications. Useful liquid dispersion media include safflower oil, ethanol, n-butanol, hexane, or glycol, among other liquids selected from known organic pharmaceutical excipients (see U.S. Patent Nos. 4,540,602 and 5,145,684), and can be present in an amount of 2.0-70%, 3-50%, or 5-25% by weight based on the total weight of the drug in the formulation.

Drug particles can also be prepared by homogeneous nucleation and precipitation in the presence of a wetting agent or dispersing agent using methods analogous to those described in U.S. Patent Nos. 5,560,932 and 5,665,331. Such a method can include the steps of: (1) dispersing drug in a suitable liquid media; (2) adding the mixture from step (1) to a mixture including at least one dispersing agent or wetting agent such that at the appropriate temperature, the drug is dissolved; and (3) precipitating the formulation from step (2) using an appropriate anti-solvent. The method can be followed by removal of any formed salt, if present, by dialysis or filtration and concentration of the dispersion by conventional means. In one embodiment, the drug particles are present in an essentially pure form and dispersed in a suitable liquid dispersion media. In this approach the drug particles are a discrete phase within the resulting mixture. Useful dispersing agents, wetting agents, solvents, and anti-solvents can be experimentally determined.

Drug particles can also be prepared by high pressure homogenization (see U.S. Patent No. 5,510,118). In this approach drug particles are dispersed in a liquid dispersion medium and subjected to repeated homogenization to reduce the size of the drug particles to the desired effective average particle size. The drug particles can be reduced in size in the presence of at least one or more dispersing agents or wetting agents. Alternatively, the drug particles can be contacted with one or more dispersing agents or wetting agents either before or after attrition. Other materials, such as a diluent, can be added to the drug/dispersing agent mixture before, during, or after the size reduction process. For example, unprocessed drug can be added to a liquid medium in which it is essentially insoluble to form a premix (i.e., about 0.1-60% w/w drug, and about 20-60% w/w dispersing agents or wetting agents). In particular embodiments, the dispersing agent is an additive of the invention (e.g., polyglycolized glycerides (e.g., Labrasol®), TPGS compounds (e.g., tocopheryl-PEG-1000-succinate, TPGS), fatty acids, fatty acid salts, fatty acid esters, carnitine esters, or a mixture thereof). The apparent viscosity of the premix suspension is preferably less than about 1000 centipoise. The premix can then be transferred to a microfluidizer and circulated continuously first at low pressures, and then at maximum capacity (i.e., 3,000 to 30,000 psi) until the desired particle size reduction is achieved. The resulting dispersion of drug particles can be included in a pharmaceutical composition of the invention.

The drug particles can be prepared with the use of one or more wetting and/or dispersing agents, which are, e.g., adsorbed on the surface of the drug particle. The drug particles can be contacted with wetting and/or dispersing agents either before, during, or after size reduction. Generally, wetting and/or dispersing agents fall into two categories: non-ionic agents and ionic agents. The most common non-ionic agents are excipients which are contained in classes known as binders, fillers, surfactants and wetting agents. Limited examples of non-ionic surface stabilizers are hydroxypropylmethylcellulose, polyvinylpyrrolidone, Plasdone, polyvinyl alcohol, Pluronics, Tweens and polyethylene glycols (PEGs). Ionic agents are typically organic molecules bearing an ionic bond such that the molecule is charged in the formulation, such as long chain sulfonic acid salts (e.g., sodium lauryl sulfate and dioctyl sodium sulfosuccinate) or fatty acid salts.

In particular embodiments, the wetting agent or dispersing agent includes an additive of the invention (e.g., polyglycolized glycerides (e.g., Labrasol®), TPGS compounds (e.g., tocopheryl-PEG-1000-succinate, TPGS), fatty acids, fatty acid salts, fatty acid esters, carnitine esters, or a mixture thereof).

Methods for making formulations for oral administration are found, for example, in "Remington: The Science and Practice of Pharmacy" (20th ed., ed. A.R. Gennaro, 2000, Lippincott Williams & Wilkins). Formulations for oral administration (e.g., tablets, pills, caplets, hard capsules, soft capsules, sachets, and liquid dosage forms) may, for example, contain any one or combination of the additives described above along with other additives and/or excipients as needed. Liquid-filled capsules can include any of the additives described herein. The capsule will contain from, for example, 10 to about 1,000 mg of cefepime, or a salt thereof. Liquid-filled capsules may, for example, contain either solutions or suspensions of cefepime, or a salt thereof, depending upon the concentration of cefepime within the capsule and the additives used in the formulation.

The formulations of the invention can also be a semi-solid formulation, e.g., solid at ambient temperature but liquid at physiological temperature. Semi-solid formulations can be made, for example, by including a sufficient amount of high molecular weight PEG (i.e., greater that 600 Da, preferably 1,500 Da) in the formulation, by varying the amount of a non-aqueous liquid (e.g., propylene glycol, N-methyl pyrrolidone, or glycerol) present in a liquid-filled capsule of the invention, or by varying the amount of gelling agent in the formulated capsule or by using a surfactant that is semi-solid at ambient temperature. Alternatively, inclusion of a surfactant having a melting point above 37 °C can result in a semi-solid formulation. Semi-solid formulations can be made, for example, by incorporating the formulation containing varying the amount of a non-aqueous liquid (e.g., propylene glycol, N-methyl pyrrolidone, or glycerol) into a sucrose matrix. Alternatively, semi-solid formulations can be made by incorporating the formulations containing varying the amount of a non-aqueous liquid (e.g., propylene glycol, N-methyl pyrrolidone, or glycerol) or formulation containing <10% w/w aqueous solution into the cefepime formulation of interest. For example, semi-solid formulations can be made by warming the TPGS to its melting point with inclusion of a surfactant or polyglycolide glyceride having a melting point above 37 °C , resulting in a semi-solid formulation. For example, a semi-sold formulation can be made by including a nonaqueous excipient in the formulation.

A particular formulation of the invention can include multiple additives (e.g., a combination two or three) to achieve not only an enhancement in oral bioavailability, but also a reduced weight percentage of additives in the formulation, allowing higher drug loadings. Thus, a combination of (i) acyl esters of carnitine and (ii) carboxylic acid, or a salt thereof, and/or an alcohol, or polyglycolized glyceride can work synergistically to increase oral absorption of the cefepime, or a pharmaceutically acceptable salt thereof, over a longer window of time and/or at a reduced overall weight percentage of additive in the formulation.

In one approach, a first amount of additive is delivered immediately form the first layer, followed by a second immediate release of the 2^{nd} additive from the inner core or second layer of a bilayer solid unit dosage form. Such delivery systems are particularly useful for tableted formulations where the components of the inner core are not in a physically ideal form for a stable tablet (e.g., where the inner core is a semi-solid, liquid, or gel).

### Therapy

The pharmaceutical compositions described herein can be used to treat or prevent bacterial infections. Bacterial infections which can be treated using the unit dosage forms of the invention include pneumonia, upper and lower respiratory tract infection, uncomplicated skin and skin structure infection, complicated skin and skin structure infection, soft tissue infection, bone and joint infection, hospital-acquired lung infection, acute bacterial otitis media, bacterial pneumonia, complicated infection, noncomplicated infection, pyelonephritis, uncomplicated intra-abdominal infection, complicated intra-abdominal infection, deep-seated abcess, bacterial sepsis, central nervous system infection, bacteremia, wound infection, peritonitis, meningitis, infections after burn, uncomplicated and complicated urinary tract infection, gastro-intestinal tract infection, pelvic inflammatory disease, endocarditis, febrile neutropenia, and intravascular infection. The formulations of the invention can specifically be used to treat any infection for which the cefepime, or a pharmaceutically acceptable salt thereof, within the formulation has been previously been reported to be an effective treatment (e.g., when administered intravenously).

Cefepime is indicated in the treatment of the following infections caused by susceptible strains of the designated microorganisms: pneumonia (moderate to severe) caused by Streptococcus pneumoniae, including cases associated with concurrent bacteremia, Pseudomonas aeruginosa, Klebsiella pneumoniae, or Enterobacter species; empiric treatment of febrile neutropenic subjects; uncomplicated and complicated urinary tract infections (including pyelonephritis) caused by Escherichia coli or Klebsiella pneumoniae, when the infection is severe, or caused by Escherichia coli, Klebsiella pneumoniae, or Proteus mirabilis, when the infection is mild to moderate, including cases associated with concurrent bacteremia with these microorganisms; uncomplicated skin and skin structure infections caused by Staphylococcus aureus (methicillin-susceptible strains only) or Streptococcus pyogenes; venereal disease caused by *Haemophilus ducreyi, Chlamydia trachomatis, Neisseria gonorrhoeae, Klebsiella granulomatis,* or *Treponema pallidum*; complicated intra-abdominal infections (used in combination with metronidazole) caused by Escherichia coli, viridans group streptococci, Pseudomonas aeruginosa, Klebsiella pneumoniae, Enterobacter, Bacteroides fragilis, or Mycobacterium spp (i.e., *Mycobacterium abscessus, M. africanum, M. agri, M. aichiense, M. alvei, M. asiaticum, M, aurum, M. austroafricanum, M. avium, M. bohemicum, M. bovis, M. branderi, M. brumae, M. celatum, M. chelonae, M. chitae, M. chlorophenolicum, M. chubuense, M. confluentis, M. conspicuum, M. cookii, M. diernhoferi, M. doricum, M. duvalii, M. elephantis, M. fallax, M. farcinogenes*, *M. flavescens*, *M. fortuitum, M. frederiksbergense, M. gadium, M. gastri, M. genavense, M. gilvum, M. goodii*, *M. gordonae, M. haemophilum, M. hassiacum, M. heckeshornense*, *M. heidelbergense, M. hiberniae*, *M. immunogenum, M. intracellulare*, *M. interjectum, M. intermedium, M. kansasii*, *M. komossense, M. kubicae, M. lentiflavum, M. leprae, M. lepraemurium, M. luteum, M. madagascariense, M. mageritense, M. malmoense, M. marinum, M. microti, M. moriokaense, M. mucogenicum, M. murale, M. neoaurum, M. nonchromogenicum, M. novocastrense, M. obuense, M. parafortuitum, M. paratuberculosis, M. peregrinum, M. phage, M. phlei, M. porcinum*, *M. poriferae, M. pulveris, M. rhodesiae, M. scrofulaceum, M. senegalense, M. septicum, M. shimoidei, M. simiae, M. smegmatis, M. sphagni, M. szulgai, M. terrae, M. thermoresistibile, M. tokaiense, M. triplex, M. triviale, M. tuberculosis, M. tusciae*, *M. ulcerans, M. vaccae, M. wolinskyi,* or *M. xenopi*).

The following examples are put forth so as to provide those of ordinary skill in the art with a complete disclosure and description of how the pharmaceutical compositions and their uses in methods as claimed herein are performed, made, and evaluated, and are intended to be purely exemplary of the invention and are not intended to be limiting. The scope of the invention is defined by the appended claims. Any of the following examples not falling within the scope of the appended claims is provided for reference purposes.

### Example 1: Liquid Cefepime formulations

The following liquid formulations can be used in the compositions and in the uses of the compositions in methods of the invention, the scope of which is defined by the appended claims.

**Table 1. Liquid Formulations**

| **Formulation** | **Composition** | **Concentration (%w/w)** |
|---|---|---|
| Formulation 1 | Palmitoyl DL Carnitine | 1.5 |
| | *Cefepime. HCL | 2.5 |
| | NaOAc, 0.1 M, pH 5.2 | 96.0 |
| | Total | 100.0 |
| | | |
| Formulation 2 | Palmitoyl DL Carnitine | 1.5 |
| | *Cefepime. HCL | 2.5 |
| | Water | 96.0 |
| | Total | 100.0 |
| | | |
| Formulation 3 | Palmitoyl DL Carnitine | 1.5 |
| | *Cefepime. HCL | 2.5 |
| | Sodium Citrate (anhydrous) | 3.0 |
| | Water | 93.0 |
| | Total | 100.0 |
| | | |
| Formulation 4 | Palmitoyl DL Carnitine | 3.9 |
| | *Cefepime. HCL | 3.9 |
| | Labrasol | 38.8 |
| | Vitamin E TPGS, USP | 2.3 |
| | NaOAc, 0.1 M, pH 5.2 | 51.2 |
| | Total | 100 |
| | | |
| Formulation 5 | Palmitoyl DL Carnitine | 6.6 |
| | *Cefepime. HCL | 6.6 |
| | Tween 80 | 4.1 |
| | Water | 82.6 |
| | Total | 100 |
| | | |
| Formulation 6 | Palmitoyl DL Carnitine | 5.3 |
| | *Cefepime. HCL | 5.3 |
| | Tween 80 | 3.3 |
| | Sodium Citrate (anhydrous) | 19.9 |
| | Water | 66.2 |
| | Total | 100.0 |
| | | |
| Formulation 7 | Palmitoyl DL Carnitine | 6.1 |
| | *Cefepime. HCL | 6.1 |
| | Tween 80 | 3.8 |
| | Sodium Citrate (anhydrous) | 7.6 |
| | Water | 76.3 |
| | Total | 100 |
| | | |
| Formulation 8 | Palmitoyl DL Carnitine | 6.5 |
| | *Cefepime. HCL | 6.5 |
| | Tween 80 | 4.1 |
| | Sodium Citrate (anhydrous) | 1.4 |
| | Water | 81.5 |
| | Total | 100.0 |
| Formulation 9 | Palmitoyl DL Carnitine | 6.5 |
| | *Cefepime. HCL | 6.5 |
| | Tween 80 | 4.1 |
| | Sodium Citrate (anhydrous) | 1.5 |
| | Water | 81.4 |
| | Total | 100.0 |
| | | |
| Formulation 10 | Palmitoyl DL Carnitine | 6.5 |
| | *Cefepime. HCL | 6.5 |
| | Tween 80 | 4.1 |
| | Sodium Citrate (anhydrous) | 1.2 |
| | Water | 81.6 |
| | Total | 100 |
| | | |
| Formulation 11 | Palmitoyl DL Carnitine | 15.7 |
| | *Cefepime. HCL | 5.2 |
| | Sodium Citrate (anhydrous) | 13.4 |
| | Water | 65.6 |
| | Total | 100 |
| | | |
| Formulation 12 | Palmitoyl DL Carnitine | 15.1 |
| | *Cefepime. HCL | 5.0 |
| | Sodium Citrate (anhydrous) | 16.7 |
| | Water | 63.1 |
| | Total | 100 |
| | | |
| Formulation 13 | Palmitoyl DL Carnitine | 6.7 |
| | *Cefepime. HCL | 3.4 |
| | Tween 80 | 4.2 |
| | Sodium Citrate (anhydrous) | 1.4 |
| | Water | 84.3 |
| | Total | 100.0 |
| | | |
| Formulation 14 | Palmitoyl DL Carnitine | 6.8 |
| | *Cefepime. HCL | 2.3 |
| | Tween 80 | 4.3 |
| | Sodium Citrate (anhydrous) | 1.4 |
| | Water | 85.2 |
| | Total | 100.0 |
| | | |
| Formulation 15 | Palmitoyl DL Carnitine | 17.3 |
| | *Cefepime. HCL | 5.8 |
| | Tween 80 | 3.6 |
| | Sodium Citrate (anhydrous) | 1.2 |
| | Water | 71.9 |
| | Total | 100 |
| Formulation 16 | Palmitoyl DL Carnitine | 15.8 |
| | *Cefepime. HCL | 5.3 |
| | Tween 80 | 10.0 |
| | Sodium tartrate (dihydrate) | 3.1 |
| | Water | 65.8 |
| | Total | 100.0 |
| | | |
| Formulation 17 | Palmitoyl DL Carnitine | 15.8 |
| | *Cefepime. HCL | 5.3 |
| | Tween 80 | 10.0 |
| | Sodium Citrate (anhydrous) | 3.1 |
| | Water | 65.8 |
| | Total | 100.0 |
| | | |
| Formulation 18 | Palmitoyl DL Carnitine | 15.4 |
| | *Cefepime. HCL | 7.7 |
| | Tween 80 | 9.8 |
| | Sodium Citrate (anhydrous) | 3.0 |
| | Water | 64.1 |
| | Total | 100.0 |
| | | |
| Formulation 19 | Palmitoyl DL Carnitine | 15.8 |
| | *Cefepime. HCL | 5.3 |
| | Tween 80 | 10.0 |
| | Sodium Lactate | 3.1 |
| | Water | 65.8 |
| | Total | 100.0 |
| | | |
| Formulation 20 | Palmitoyl DL Carnitine | 17.5 |
| | *Cefepime. HCL | 5.8 |
| | Sodium tartrate (dihydrate) | 3.6 |
| | Water | 73.0 |
| | Total | 100 |
| | | |
| Formulation 21 | Palmitoyl DL Carnitine | 16.6 |
| | *Cefepime. HCL | 5.5 |
| | Tween 80 | 5.2 |
| | Sodium tartrate (dihydrate) | 3.5 |
| | Water | 69.2 |
| | Total | 100.0 |
| | | |
| Formulation 22 | Palmitoyl DL Carnitine | 16.3 |
| | *Cefepime. HCL | 5.4 |
| | Propylene Glycol | 6.8 |
| | Sodium tartrate (dihydrate) | 3.4 |
| | Water | 68.0 |
| | Total | 100.0 |
| Formulation 23 | Palmitoyl DL Carnitine | 16.9 |
| | *Cefepime. HCL | 5.6 |
| | Glycerin | 14.1 |
| | Sodium tartrate (dihydrate) | 3.5 |
| | Water | 59.9 |
| | Total | 100.0 |
| | | |
| Formulation 24 | Palmitoyl DL Carnitine | 17.5 |
| | *Cefepime. HCL | 5.8 |
| | Tween 40 | 10.9 |
| | Sodium tartrate (dihydrate) | 3.6 |
| | Water | 62.0 |
| | Total | 100 |
| | | |
| Formulation 25 | Palmitoyl DL Carnitine | 25.7 |
| | *Cefepime. HCL | 6.4 |
| | Sodium tartrate (dihydrate) | 3.5 |
| | Water | 64.3 |
| | Total | 100 |
| | | |
| Formulation 26 | Palmitoyl DL Carnitine | 25.7 |
| | *Cefepime. HCL | 6.4 |
| | Glycerin | 12.9 |
| | Sodium tartrate (dihydrate) | 3.5 |
| | Water | 51.4 |
| | Total | 100 |
| | | |
| Formulation 27 | Palmitoyl DL Carnitine | 30.0 |
| | *Cefepime. HCL | 7.5 |
| | Sodium tartrate (dihydrate) | 6.3 |
| | Water | 56.3 |
| | Total | 100 |
| | | |
| Formulation 28 | Palmitoyl DL Carnitine | 30.0 |
| | *Cefepime. HCL | 7.5 |
| | Glycerin | 12.5 |
| | Sodium tartrate (dihydrate) | 6.3 |
| | Water | 43.8 |
| | Total | 100 |
| | | |
| Formulation 29 | Palmitoyl DL Carnitine | 25.1 |
| | *Cefepime. HCL | 8.4 |
| | Sodium tartrate (dihydrate) | 3.8 |
| | Water | 62.7 |
| | Total | 100.0 |
| Formulation 30 | Palmitoyl DL Carnitine | 24.2 |
| | *Cefepime. HCL | 8.1 |
| | Glycerin | 10.1 |
| | Sodium tartrate (dihydrate) | 3.7 |
| | Water | 53.9 |
| | Total | 100.0 |
| | | |
| Formulation 31 | Palmitoyl DL Carnitine | 22.9 |
| | *Cefepime. HCL | 7.6 |
| | Sodium tartrate (dihydrate) | 4.6 |
| | Water | 64.9 |
| | Total | 100.0 |
| | | |
| Formulation 32 | Palmitoyl DL Carnitine | 22.9 |
| | *Cefepime. HCL | 7.6 |
| | Glycerin | 11.5 |
| | Sodium tartrate (dihydrate) | 4.6 |
| | Water | 53.4 |
| | Total | 100.0 |
| | | |
| Formulation 33 | Palmitoyl DL Carnitine | 22.8 |
| | *Cefepime. HCL | 7.6 |
| | Sodium tartrate (dihydrate) | 4.6 |
| | Sodium Chloride | 0.3 |
| | Water | 64.7 |
| | Total | 100 |
| | | |
| Formulation 34 | Palmitoyl DL Carnitine | 22.8 |
| | *Cefepime. HCL | 7.6 |
| | Glycerin | 11.4 |
| | Sodium tartrate (dihydrate) | 4.6 |
| | Sodium Chloride | 0.3 |
| | Water | 53.3 |
| | Total | 100.0 |
| | | |
| Formulation 35 | Palmitoyl DL Carnitine | 22.9 |
| | *Cefepime. HCL | 7.6 |
| | Polyethylene Glycol 400 | 19.1 |
| | Sodium tartrate (dihydrate) | 4.6 |
| | Water | 45.8 |
| | Total | 100.0 |
| | | |
| Formulation 36 | Palmitoyl DL Carnitine | 22.1 |
| | *Cefepime. HCL | 7.4 |
| | Glycerin | 33.1 |
| | Sodium tartrate (dihydrate) | 4.4 |
| | Water | 33.1 |
| | Total | 100 |
| Formulation 37 | Palmitoyl DL Carnitine | 22.9 |
| | *Cefepime. HCL | 7.6 |
| | Labrafil M 1944 CS | 45.8 |
| | Sodium tartrate (dihvdrate) | 4.6 |
| | Water | 19.1 |
| | Total | 100.0 |
| | | |
| Formulation 38 | Palmitoyl DL Carnitine | 20.1 |
| | *Cefepime. HCL | 6.7 |
| | Sucrose (Dipac) | 14.4 |
| | Sodium tartrate (dihvdrate) | 4.0 |
| | Water | 54.7 |
| | Total | 100 |
| | | |
| Formulation 39 | Palmitoyl DL Carnitine | 21.2 |
| | *Cefepime. HCL | 7.1 |
| | Sucrose (Dipac) | 7.1 |
| | Sodium tartrate (dihydrate) | 4.2 |
| | Sodium Chloride | 0.3 |
| | Water | 60.1 |
| | Total | 100.0 |
| | | |
| Formulation 40 | Palmitoyl DL Carnitine | 22.9 |
| | *Cefepime. HCL | 7.6 |
| | Propylene Glycol | 19.1 |
| | Sodium tartrate (dihydrate) | 4.6 |
| | Water | 45.8 |
| | Total | 100.0 |
| | | |
| Formulation 41 | Palmitoyl DL Carnitine | 21.3 |
| | *Cefepime. HCL | 7.1 |
| | Propylene Glycol | 19.7 |
| | Sodium tartrate (dihydrate) | 4.7 |
| | Water | 47.2 |
| | Total | 100.0 |
| | | |
| Formulation 42 | Palmitoyl DL Carnitine | 19.9 |
| | *Cefepime. HCL | 6.6 |
| | Sodium tartrate (dihydrate) | 7.4 |
| | Water | 66.2 |
| | Total | 100 |
| | | |
| Formulation 43 | Palmitoyl DL Carnitine | 20.7 |
| | *Cefepime. HCL | 6.9 |
| | Sodium tartrate (dihydrate) | 10.3 |
| | Water | 55.9 |
| | Total | 100.0 |
| Formulation 44 | Palmitoyl DL Carnitine | 20.6 |
| | *Cefepime. HCL | 6.9 |
| | Propylene Glycol | 64.9 |
| | Sodium tartrate (dihydrate) | 7.6 |
| | Total | 100.0 |
| | | |
| Formulation 45 | Palmitoyl DL Carnitine | 20.1 |
| | *Cefepime. HCL | 6.7 |
| | Propylene Glycol | 7.4 |
| | Sodium tartrate (dihydrate) | 7.4 |
| | Sodium chloride | 0.3 |
| | Water | 58.0 |
| | Total | 100 |
| | | |
| Formulation 46 | Palmitoyl DL Carnitine | 20.9 |
| | *Cefepime. HCL | 7.0 |
| | Propylene Glycol | 3.9 |
| | Sodium tartrate (dihydrate) | 4.6 |
| | Sodium chloride | 0.3 |
| | Water | 63.3 |
| | Total | 100.0 |
| | | |
| Formulation 47 | Palmitovl DL Carnitine | 20.7 |
| | *Cefepime. HCL | 6.9 |
| | Propylene Glycol | 7.7 |
| | Sodium tartrate (dihydrate) | 4.6 |
| | Sodium chloride | 0.3 |
| | Water | 59.8 |
| | Total | 100.0 |
| | | |
| Formulation 48 | Palmitoyl DL Carnitine | 19.1 |
| | *Cefepime. HCL | 6.4 |
| | Vitamin E TPGS | 7.1 |
| | Sodium tartrate (dihydrate) | 4.3 |
| | Tween 80 | 2.8 |
| | Water | 60.3 |
| | Total | 100.0 |
| | | |
| Formulation 49 | Palmitoyl DL Carnitine | 20.5 |
| | *Cefepime. HCL | 6.8 |
| | Sucrose (Dipac) | 6.8 |
| | Propylene Glycol | 3.4 |
| | Sodium tartrate (dihydrate) | 4.1 |
| | Sodium chloride | 0.3 |
| | Water | 58.0 |
| | Total | 100 |
| Formulation 50 | Palmitovl DL Carnitine | 18.7 |
| | *Cefepime. HCL | 6.2 |
| | Propylene Glycol | 3.9 |
| | Sodium tartrate (dihydrate) | 4.7 |
| | Sodium chloride | 0.4 |
| | Water | 66.2 |
| | Total | 100 |
| | | |
| Formulation 51 | Palmitoyl DL Carnitine | 18.0 |
| | *Cefepime. HCL | 6.0 |
| | Sucrose (Dipac) | 7.5 |
| | Sodium tartrate (dihvdrate) | 4.5 |
| | Sodium Chloride | 0.3 |
| | Water | 63.7 |
| | Total | 100.0 |
| | | |
| Formulation 52 | Palmitoyl DL Carnitine | 18.1 |
| | *Cefepime. HCL | 6.0 |
| | Sucrose (Dipac) | 5.3 |
| | Propylene Glycol | 3.8 |
| | Sodium tartrate (dihvdrate) | 4.5 |
| | Sodium chloride | 0.3 |
| | Water | 61.9 |
| | Total | 100 |
| | | |
| Formulation 53 | Palmitoyl DL Carnitine | 19.5 |
| | *Cefepime. HCL | 6.5 |
| | Sodium Chloride | 0.7 |
| | Water | 73.2 |
| | Total | 100 |
| | | |
| Formulation 54 | Palmitoyl DL Carnitine | 19.2 |
| | *Cefepime. HCL | 6.4 |
| | Sodium tartrate (dihydrate) | 2.4 |
| | Water | 72.0 |
| | Total | 100.0 |
| | | |
| Formulation 55 | Palmitoyl DL Carnitine | 19.6 |
| | *Cefepime. HCL | 6.5 |
| | Sodium Chloride | 0.4 |
| | Water | 73.5 |
| | Total | 100 |
| | | |
| Formulation 56 | Palmitoyl DL Carnitine | 19.2 |
| | *Cefepime. HCL | 6.4 |
| | Ethanol (200 proof) | 12.0 |
| | Sodium tartrate (dihydrate) | 2.4 |
| | Water | 60.0 |
| | Total | 100.0 |
| Formulation 57 | Palmitoyl DL Carnitine | 19.7 |
| | *Cefepime. HCL | 6.6 |
| | Hydrochloric Acid (36%) | 8.2 |
| | Water | 65.6 |
| | Total | 100 |
| | | |
| Formulation 58 | Palmitoyl DL Carnitine | 19.7 |
| | *Cefepime. HCL | 6.6 |
| | Ethanol (200 proof) | 16.4 |
| | Water | 57.4 |
| | Total | 100 |
| | | |
| Formulation 59 | Palmitoyl DL Carnitine | 18.8 |
| | *Cefepime. HCL | 6.3 |
| | Sucrose (Dipac) | 3.9 |
| | Sodium tartrate (dihydrate) | 4.3 |
| | Sodium chloride | 0.4 |
| | Water | 66.4 |
| | Total | 100 |
| | | |
| Formulation 60 | Palmitoyl DL Carnitine | 19.1 |
| | *Cefepime. HCL | 6.4 |
| | Propylene Glycol | 2.0 |
| | Sodium tartrate (dihydrate) | 4.4 |
| | Sodium chloride | 0.4 |
| | Water | 67.8 |
| | Total | 100 |

| | | |
|---|---|---|
| *as free base | | |

### Example 2: Solid Cefepime formulations

The following solid formulations can be used in the compositions and in the uses of the compositions in methods of the invention, the scope of which is defined by the appended claims.

**Table 2: Solid tablet and semi-solid dosages**

| **Formulation** | **Composition** | **weight** |
|---|---|---|
| Formulation 61 Tablet | Palmitoyl DL Carnitine | 200mg |
| | *Cefepime. HCL | 200mg |
| | Mannitol | 480mg |
| | disintegrant | 20mg |
| | Total | 900mg |
| | | |
| Formulation 62 Semi-solid in capsule | Palmitoyl DL Carnitine | 250mg |
| | *Cefepime. HCL | 250mg |
| | Labrasol | 600mg |
| | Cellulose | 150mg |
| | Total | 1250mg |
| | | |

| | | |
|---|---|---|
| *as free base | | |

**Table 3: Dry blend formulations for reconstitution**

| **Formulation** | **Composition** | **Concentration (%w/w)** |
|---|---|---|
| Formulation B1 | Palmitoyl DL Carnitine | 59.3 |
| | *Cefepime. HCL | 19.8 |
| | Propylene Glycol | 6.2 |
| | Sodium Tartrate (dihydrate) | 13.6 |
| | Sodium Chloride | 1.1 |
| | Total | 100.0 |
| | | |
| Formulation B2 | Palmitoyl DL Carnitine | 55.9 |
| | *Cefepime. HCL | 18.6 |
| | Sucrose (Dipac) | 11.6 |
| | Sodium Tartrate (dihydrate) | 12.8 |
| | Sodium Chloride | 1.0 |
| | Total | 100 |
| | | |
| Formulation B3 | Palmitoyl DL Carnitine | 61.49 |
| | *Cefepime. HCL | 20.49 |
| | Sodium Tartrate (dihydrate) | 14.08 |
| | Total | 100 |
| | | |
| Formulation B4 | Palmitoyl DL Carnitine | 71.52 |
| | *Cefepime. HCL | 23.87 |
| | Total | 100 |
| | | |
| Formulation B5 | Palmitoyl DL Carnitine | 52.72 |
| | Cefepime. coated | |
| | *Cefepime. HCL | 17.57 |
| | Eudragit polymer (Eudragit L100) | 17.57 |
| | Sodium Tartrate (dihydrate | 12.17 |
| | Total | 100 |
| Formulation B6 | Palmitoyl DL Carnitine | 50 |
| | Cefepime coated | |
| | *Cefepime. HCL | 12.5 |
| | Stearic acid | 37.5 |
| | Total | 100 |

| | | |
|---|---|---|
| *as free base | | |

### Example 3: Oral dosage of cefepime in canine subjects

The objective of this study was to assess the pharmacokinetic profiles of cefepime in the formulations of the invention following oral administration in male beagle dogs.

The animals were fasted at least 12 hours prior to each dose and offered feed after the 4-hour blood sample has been taken. Water was withheld for 1 hour prior to and 4 hours following each dosing event. Each dosing event was followed by a ~20 mL dose of water. Each dosing event was separated by a minimum 3 day washout period. The dose for each animal was based on the most recent body weight.

Blood was collected from the animals for pharmacokinetic analysis. Approximately 1 mL of blood was collected in K₃EDTA tubes via the jugular (or other suitable vein). For each dosing event, blood was collected at predetermined timepoints (e.g., at 15 minutes, 30 minutes, 45 minutes, 60 minutes, 90 minutes, 2 hours, 3 hours, and 4 hours) following dosing.

Following blood collection, the samples were kept on ice and centrifuged (within ∼30 minutes of collection) under refrigeration (∼5 °C for ∼10 minutes at ∼2000 x g). The cellular components and plasma were separated. Cellular components were discarded. The plasma was harvested into a single tube for each animal at each timepoint and stored frozen at approximately -70 °C and later analyzed for cefepime concentration.

The peak plasma concentration (Cₘₐₓ), the time required to achieve the peak plasma concentration (Tₘₐₓ), and the area under the plasma concentration time curve (AUC) was calculated from the plasma concentration data. The plasma terminal half-life (T_{1/2}) was estimated if possible. Oral bioavailability was calculated by comparison of the circulating concentrations to those observed for intravenously administered drug. All pharmacokinetic calculations were done using WinNonlin version 4.1 (Pharsight Corp) by non-compartmental analysis.

Compound/formulation combinations were prepared fresh on the day of dosing by dissolving or suspending the compound in the formulations. The formulations were used within 1 - 2 hours of preparation. The formulation details for cefepime, as well as their resulting oral bioavailability, are given in Table 4.

**Table 4.**

| **Formulation** | **T_{1/2} (Minutes)** | **Oral Bioavailability Average** |
|---|---|---|
| 1 | 70 | 14% |
| 4 | 83 | 22% |
| saline | 104 | 7.8% |

The PK curve observed for formulation 4 is depicted in Figure 1, along with the curve for cefepime administered intravenously (10 mg/kg). The PK curves observed for formulation 1 and for cefepime in saline are depicted in Figure 2.

### Example 4: Formulations for reconstitution

The following tables (Tables 4a-4g) describe oral dosage forms that can be used in the compositions and in the uses of the compositions in methods of the invention, the scope of which is defined by the appended claims.

**Table 4a. Reconstitutable Formulation 65.**

| **Ingredients** | **% w/v** |
|---|---|
| Palmitoyl DL Carnitine | 17.8 |
| *Cefepime. HCL | 5.9 |
| Sucrose (Dipac) | 12.6 |
| Sodium tartrate | 4.1 |
| Sodium chloride | 0.3 |
| Acesulfamate K | 1.5 |
| Sucralose | 0.7 |
| Artificial Strawberry and Cream Flavor | 0.9 |
| Artificial Banana Flavor | 0.6 |
| Water | 55.6 |
| Total | 100 |

| | |
|---|---|
| *as free base | |

**Table 4b. Reconstitutable Formulation 66.**

| **Ingredients** | **% w/v** |
|---|---|
| **Active Component** | |
| *Cefepime. HCL | 5.9 |

| **Placebo Component** | |
|---|---|
| Palmitoyl DL Carnitine | 17.8 |
| Sucrose (Dipac) | 12.6 |
| Sodium tartrate | 4.1 |
| Sodium chloride | 0.3 |
| Acesulfamate K | 1.5 |
| Sucralose | 0.7 |
| Artificial Strawberry and Cream Flavor | 0.9 |
| Artificial Banana Flavor | 0.6 |

| **For Reconstitution** | |
|---|---|
| Water | 55.6 |
| | |
| Total | 100 |

| | |
|---|---|
| *as free base | |

**Table 4c. Reconstitutable Formulation 67.**

| **Ingredients** | **% w/v** |
|---|---|
| Palmitoyl DL Carnitine | 17.8 |
| *Cefepime. HCL | 5.9 |
| Propylene Glycol | 1.9 |
| Sucrose (Dipac) | 10.7 |
| Sodium tartrate | 4.1 |
| Sodium chloride | 0.3 |
| Acesulfamate K | 1.5 |
| Sucralose | 0.7 |
| Artificial Strawberry and Cream Flavor | 0.9 |
| Artificial Banana Flavor | 0.6 |
| Water | 55.6 |
| Total | 100 |

| | |
|---|---|
| *as free base | |

**Table 4d. Reconstitutable Formulation 68.**

| **Ingredients** | **% w/v** |
|---|---|
| **Active Component** | |
| *Cefepime. HCL | 5.9 |

| **Placebo Component** | |
|---|---|
| Propylene Glycol | 1.9 |
| Palmitoyl DL Carnitine | 17.8 |
| Sucrose (Dipac) | 10.7 |
| Sodium tartrate | 4.1 |
| Sodium chloride | 0.3 |
| Acesulfamate K | 1.5 |
| Sucralose | 0.7 |
| Artificial Strawberry and Cream Flavor | 0.9 |
| Artificial Banana Flavor | 0.6 |

| **For Reconstitution** | |
|---|---|
| Water | 55.6 |
| | |
| Total | 100 |

| | |
|---|---|
| *as free base | |

**Table 4e. Reconstitutable Formulation 69.**

| **Ingredients** | **% w/v** |
|---|---|
| **Active Component** | |
| *Cefepime. HCL | 19.8 |

| **Placebo Component** | |
|---|---|
| Propylene Glycol | 5.2 |
| Palmitoyl DL Carnitine | 59.3 |
| Sodium tartrate | 12.6 |
| Sodium chloride | 1.1 |
| Artificial sweetening agents mix | 1.0 |
| Artificial Strawberry and Cream Flavor | 0.9 |
| Artificial Banana Flavor | 0.6 |
| Total | 100 |

| | |
|---|---|
| *as free base | |

**Table 4f. Reconstitutable Formulation 70.**

| **Ingredients** | **% w/v** |
|---|---|
| Palmitoyl DL Carnitine | 22.9 |
| *Cefepime. HCL | 7.6 |
| Eudragit Polymer (Eudragit L100) | 7.6 |
| Sodium tartrate | 4.6 |

| **For Reconstitution** | |
|---|---|
| Water | 57.25 |
| Total | 100 |

| | |
|---|---|
| *as free base | |

**Table 4g. Reconstitutable Formulation 71.**

| **Ingredients** | **% w/v** |
|---|---|
| Palmitoyl DL Carnitine | 20.69 |
| *Cefepime. HCL | 6.8 |
| Stearic acid | 20.69 |

| **For Reconstitution** | |
|---|---|
| Water | 51.72 |
| Total | 100 |

| | |
|---|---|
| *as free base | |

The reconstitutable formulations can be provided, for example, as sachets which are cut opened and the solid contents of the sachets are combined with a predetermined volume of water prior to oral administration. For certain formulations the active and inactive components of the formulation may be provided separately (i.e., as separate packets) and combined with water just prior to administration.

### Example 5: Stability of Cefepime Formulation 72.

The stability of cefepime formulated as a dry blend (Formulation 72) was monitored by HPLC. Formulation 72 was prepared by mixing cefepime with all other excipients listed in Table 5a and stored in the dark at room temperature. The dry blend was reconstituted with water prior to analysis.

**Table 5a. Formulation 72.**

| **COMPONENT:** | **AMOUNT PER 1G DOSE (G)** | **PERCENTAGE (%w/w)** |
|---|---|---|
| Cefepime. HCl* | *1.000 | *13.6 |
| PEG 400 Decanoate Esters | 4.002 | 45.6 |
| Tween 80, NF | 0.600 | 6.8 |
| Lemon Oil, Cold Pressed, California Type USP | 0.138 | 1.6 |
| Acesulfamate K (Sunnett® Pharma D) | 0.305 | 3.5 |
| Emprove® Sucralose Powder NF | 0.179 | 2.0 |
| Compressible Sugar DiPac, NF (Sucrose) | 1.536 | 17.5 |
| Sodium Citrate, anhydrous, NF | 0.395 | 4.5 |
| Strawberries and Cream Flavor, artificial USP | 0.250 | 2.8 |
| Banana flavor, artificial USP | 0.180 | 2.0 |
| **Total** | **8.783** | **100.0** |

| | | |
|---|---|---|
| * adjusted for assay and moisture | | |

For each time point the dry blend formulation was reconstituted with water and tested to determine the percentage of cefepime remaining in the formulation. The results are provided in Table 5b.

**Table 5b. Stability Data for Formulation 72.**

| **Time Point** | **CP-001 Content (% w/w)** | **Nominal (%) (Change from Initial)** |
|---|---|---|
| Initial | 7.30 | 100.0 |
| 2 Days | 7.21 | 98.8 |
| 7 Days | 6.70 | 91.8 |
| 10 Days | 6.62 | 90.7 |

Formulation 72 was found to be stable only for a minimum of 2 days at room temperature.

### Example 6: Stability of Cefepime Formulation 73.

The stability of cefepime formulated as a dry blend (Formulation 73) was monitored by HPLC. Formulation 73 was prepared by mixing cefepime with all other excipients listed in Table 6a and stored in the dark at room temperature. The dry blend was reconstituted with water prior to analysis.

**Table 6a. Formulation 73.**

| **Component** | **Percentage (% w/w)** |
|---|---|
| Cefepime HCl | 12.9 |
| Labrasol® | 66.9 |
| Vitamin E TPGS | 16.7 |
| Sodium Chloride | 0.45 |
| Sodium Citrate, Dihydrate | 3.1 |

For each time point the dry blend formulation was reconstituted with water and tested to determine the percentage of cefepime remaining in the formulation. The results are provided in Table 6b.

**Table 6b. Stability Data for Formulation 73.**

| **Time Point** | **Cefepime Content (%w/w)** | **% Nominal (Based on Initial Result)** |
|---|---|---|
| Initial | 11.15 | 100.0 |
| 1 Day | 11.32 | 101.5 |
| 2 Day | 11.39 | 102.2 |
| 3 Day | 10.26 | 92.0 |

Formulation 73 was found to be stable only for a minimum of 2 days at room temperature.

### Example 7: Stability of Cefepime Formulation 74.

The stability of cefepime formulated as a dry blend (Formulation 74) was monitored by HPLC. Formulation 74 was prepared by mixing cefepime with all other excipients listed in Table
7a and stored in the dark at room temperature. The dry blend was reconstituted with water prior to analysis.

**Table 7a. Formulation 74.**

| Ingredients | Composition by weight |
|---|---|
| Cefepime HCL | 250mg (base) |
| Labrasol, USP/EUP | 600mg |
| Na caprate | 125mg |

For each time point the dry blend formulation was reconstituted with water and tested to determine the percentage of cefepime remaining in the formulation. The results are provided in Table 7b.

**Table 7b. Stability Data for Formulation 74.**

| Time Point | Cefepime Content (mg/Capsule Fill Weight) | % Nominal *(Based on Initial Result)* |
|---|---|---|
| Initial | 250.34 | 100.0 |
| 1 Week | 198.32 | 79.2 |
| 2 Weeks | 187.14 | 74.8 |

Formulation 74 was found to be unstable after 1 week at room temperature.

### Example 8: Stability of Cefepime Formulation 75.

The stability of cefepime formulated with palmitoyl carnitine as a dry blend (Formulation 75) was monitored by HPLC. Formulation 75 was prepared by mixing cefepime with all other excipients listed in Table 8a and stored in the dark at room temperature. The dry blend was reconstituted with water prior to analysis.

**Table 8a. Formulation 75.**

| **Ingredients** | **Amount (mg)** | **% w/w** |
|---|---|---|
| Cefepime HCl* | 80* | 23.87 |
| Palmitoyl Carnitine | 240 | 71.52 |

| | | |
|---|---|---|
| *adjusted for assay and moisture | | |

For each time point the dry blend formulation was reconstituted with water and tested to determine the percentage of cefepime remaining in the formulation. The results are provided in Table 8b.

**Table 8b. Stability Data for Formulation 75.**

| **Time Point** | **Cefepime Content (% w/w)** | **% Label Claim** | **% Nominal of Initial Result** |
|---|---|---|---|
| Initial | 23.00 | 96.34 | 100.0 |
| 1 month | 23.70 | 99.29 | 103.0 |
| 6 months | 22.35 | 93.65 | 97.2 |

Formulation 75 was found to be stable for several months at room temperature.

### Example 9: Stability of Cefepime Formulation 76.

The stability of cefepime formulated with palmitoyl carnitine as a dry blend (Formulation 76) was monitored by HPLC. Formulation 76 was prepared by mixing cefepime with all other excipients listed in Table 9a and stored in the dark at room temperature. The dry blend was reconstituted with water prior to analysis.

**Table 9a. Formulation 76.**

| **Ingredients** | **Amount (mg)** | **% w/w** |
|---|---|---|
| Cefepime HCl* | 80* | 20.49 |
| Palmitovl Carnitine | 240 | 61.44 |
| Sodium Tartrate | 55 | 14.08 |

| | | |
|---|---|---|
| *adjusted for assay and moisture | | |

For each time point the dry blend formulation was reconstituted with water and tested to determine the percentage of cefepime remaining in the formulation. The results are provided in Table 9b.

**Table 9b. Stability Data for Formulation 76.**

| **Time Point** | **Cefepime Content (% w/w)** | **% Nominal of Initial Result** |
|---|---|---|
| Initial | 20.66 | 100.0 |
| 3 months | 21.31 | 103.1 |
| 6 months | 21.02 | 101.7 |

Formulation 76 was found to be stable for at least 6 months at room temperature.

The results from Examples 8 and 9 demonstrate that cefepime is compatible with acyl carnitines, such as palmitoyl carnitine, in a dry blend powder form and that formulations with an acyl carnitine can be developed into a finished pharmaceutical form having adequate commercial shelf-life.

## Claims

1. A pharmaceutical composition in oral dosage form comprising cefepime, or a pharmaceutically acceptable salt thereof, and an additive comprising an acyl carnitine or a salt thereof, wherein said additive is present in an amount sufficient to increase the oral bioavailability of said cefepime, or said pharmaceutically acceptable salt thereof,
wherein said oral dosage form comprises a (w/w) ratio of cefepime, or a pharmaceutically acceptable salt thereof, to acyl carnitine of from 1:0.5 to 1:6.

2. The pharmaceutical composition of claim 1, wherein said oral dosage form comprises a (w/w) ratio of cefepime, or a pharmaceutically acceptable salt thereof, to acyl carnitine of from 1:2 to 1:5.

3. The pharmaceutical composition of any one of claims 1 or 2, wherein said additive further comprises an alcohol, a polysorbate surfactant, a carboxylic acid, a polyethylene glycol, a polyglycolized glyceride, alkyl saccharides, ester saccharides, a TPGS compound, or a sugar.

4. The pharmaceutical composition of claim 3:
wherein said unit dosage form comprises from 1% to 60% (w/w) of an alcohol selected from propylene glycol, ethanol, or glycerol;
wherein said unit dosage form comprises from 1% to 10% (w/w) of a carboxylic acid selected from citric acid, succinic acid, tartaric acid, fumaric acid, maleic acid, malonic acid, glutaric acid, adipic acid, lactic acid, malic acid, L-glutamic acid, L-aspartic acid, gluconic acid, glucuronic acid, salicylic acid, and salts thereof;
wherein said unit dosage form comprises from 0.5% to 10% (w/w) of a polysorbate surfactant;
wherein said unit dosage form comprises from 0.2% to 12% (w/w) TPGS compound;
wherein said unit dosage form comprises from 2% to 30% (w/w) polyethylene glycol;
wherein said unit dosage form comprises from 5% to 30% (w/w) alkyl saccharide or ester saccharide;
wherein said unit dosage form comprises from 5% to 60% (w/w) polyglycolized glyceride; or
wherein said unit dosage form comprises from 5% to 25% (w/w) sugar selected from monosaccharides, disaccharides, and sugar alcohols.

5. The pharmaceutical composition of any of claims 1-4, wherein said acyl carnitine is selected from oleoyl carnitine, palmitoyl carnitine, decanoyl carnitine, dodecanoyl carnitine, myristoyl carnitine, stearoyl carnitine, and salts thereof.

6. The pharmaceutical composition of any one of claims 1-5:
wherein said unit dosage form is formulated as a liquid-filled gel capsule or as a sachet for reconstitution; or
wherein said unit dosage form comprises a solid or semisolid, such as a powder.

7. The pharmaceutical composition of any of claims 1-6, wherein said oral dosage form comprises from 5% to 50% (w/w) acyl carnitine or a pharmaceutically acceptable salt thereof.

8. The pharmaceutical composition of claim 7:
wherein said pharmaceutical composition is a liquid dosage form comprising from 1.5% to 15% (w/w) cefepime or a pharmaceutically acceptable salt thereof;
wherein said pharmaceutical composition is a solid or semisolid dosage form comprising from 10% to 33% (w/w) cefepime or a pharmaceutically acceptable salt thereof; or
wherein said pharmaceutical composition is a tablet comprising a disintegrant and a hydrophilic matrix, optionally selected from maltose, mannitol, sucrose, crystalline cellulose, cornstarch, and methylcellulose.

9. The pharmaceutical composition of any of claims 1-5, wherein said unit dosage form comprises from 0.1% to 60% (w/w) water, more specifically from 0.1% (w/w) to 15% (w/w) water, optionally:
wherein said unit dosage form is formulated as a suspension for reconstitution; or
wherein said unit dosage form is formulated as a liquid-filled gel capsule.

10. The pharmaceutical composition of any of claims 1-5, wherein said cefepime, or a pharmaceutically acceptable salt thereof, and said additive are adsorbed onto a solid matrix.

11. The pharmaceutical composition of claim 10, wherein said solid matrix is a porous silicate, optionally:
wherein said porous silicate comprises aluminum silicate, magnesium aluminum silicate, sodium silicate, potassium silicate, magnesium silicate or calcium silicate; or
wherein said pharmaceutical composition comprises from 0.2% to 5% (w/w) chloride salt.

12. The pharmaceutical composition of claim 1, wherein said pharmaceutical composition is a reconstitutable blend comprising from 50% (w/w) to 75% (w/w) acyl carnitine and from 15% (w/w) to 25% (w/w) cefepime, or a salt thereof.

13. The pharmaceutical composition of claim 12:
wherein said pharmaceutical composition further comprises from 10% (w/w) to 20% (w/w) carboxylic acid or a salt thereof; and/or
wherein said pharmaceutical composition is a powder.

14. A pharmaceutical composition according to any one of claims 1-13 for use in a method of treating a bacterial infection in a subject, said method comprising orally administering to said subject a pharmaceutical composition of any of claims 1 to 13, wherein said composition is administered in an amount effective to treat said infection.

## Patentansprüche

1. Pharmazeutische Zusammensetzung in oraler Dosierungsform, umfassend Cefepim oder ein pharmazeutisch unbedenkliches Salz davon und einen Acylcarnitin oder ein Salz davon umfassenden Zusatz, wobei der Zusatz in einer Menge vorliegt, die ausreicht, um die orale biologische Verfügbarkeit des Cefepims oder des pharmazeutisch unbedenklichen Salzes davon zu erhöhen,
wobei die orale Dosierungsform ein Gewichtsverhältnis von Cefepim oder einem pharmazeutisch unbedenklichen Salz davon zu Acylcarnitin von 1:0,5 bis 1:6 umfasst.

2. Pharmazeutische Zusammensetzung nach Anspruch 1, wobei die orale Dosierungsform ein Gewichtsverhältnis von Cefepim oder einem pharmazeutisch unbedenklichen Salz davon zu Acylcarnitin von 1:2 bis 1:5 umfasst.

3. Pharmazeutische Zusammensetzung nach einem der Ansprüche 1 oder 2, wobei der Zusatz weiterhin einen Alkohol, ein Polysorbattensid, eine Carbonsäure, einen Polyethylenglykol, ein polyglykolysiertes Glycerid, Alkylsaccharide, Estersaccharide, eine TPGS-Verbindung oder einen Zucker umfasst.

4. Pharmazeutische Zusammensetzung nach Anspruch 3:
wobei die Einheitsdosisform 1 Gew.-% bis 60 Gew.-% eines aus Propylenglykol, Ethanol und Glycerin ausgewählten Alkohols umfasst,
wobei die Einheitsdosisform 1 Gew.-% bis 10 Gew.-% einer aus Citronensäure, Bernsteinsäure, Weinsäure, Fumarsäure, Maleinsäure, Malonsäure, Glutarsäure, Adipinsäure, Milchsäure, Äpfelsäure, L-Glutaminsäure, L-Asparaginsäure, Gluconsäure, Glucuronsäure, Salicylsäure und Salzen davon ausgewählten Carbonsäure umfasst,
wobei die Einheitsdosisform 0,5 Gew.-% bis 10 Gew.-% eines Polysorbattensids umfasst,
wobei die Einheitsdosisform 0,2 Gew.-% bis 12 Gew.-% einer TPGS-Verbindung umfasst,
wobei die Einheitsdosisform 2 Gew.-% bis 30 Gew.-% Polyethylenglykol umfasst,
wobei die Einheitsdosisform 5 Gew.-% bis 30 Gew.-% Alkylsaccharid oder Estersaccharid umfasst,
wobei die Einheitsdosisform 5 Gew.-% bis 60 Gew.-% polyglykolysiertes Glycerid umfasst oder
wobei die Einheitsdosisform 5 Gew.-% bis 25 Gew.-% aus Monosacchariden, Disacchariden und Zuckeralkoholen ausgewählten Zucker umfasst.

5. Pharmazeutische Zusammensetzung nach einem der Ansprüche 1-4, wobei das Acylcarnitin aus Oleoylcarnitin, Palmitoylcarnitin, Decanoylcarnitin, Dodecanoylcarnitin, Myristoylcarnitin, Stearoylcarnitin und Salzen davon ausgewählt ist.

6. Pharmazeutische Zusammensetzung nach einem der Ansprüche 1-5:
wobei die Einheitsdosisform als flüssigkeitsgefüllte Gelkapsel oder als Beutel zur Rekonstitution formuliert ist oder
wobei die Einheitsdosisform einen Feststoff oder einen halbfesten Stoff wie ein Pulver umfasst.

7. Pharmazeutische Zusammensetzung nach einem der Ansprüche 1-6, wobei die orale Dosierungsform 5 Gew.-% bis 50 Gew.-% Acylcarnitin oder ein pharmazeutisch unbedenkliches Salz davon umfasst.

8. Pharmazeutische Zusammensetzung nach Anspruch 7:
wobei es sich bei der pharmazeutischen Zusammensetzung um eine flüssige Dosierungsform handelt, die 1,5 Gew.-% bis 15 Gew.-% Cefepim oder eines pharmazeutisch unbedenklichen Salzes davon umfasst,
wobei es sich bei der pharmazeutischen Zusammensetzung um eine feste oder halbfeste Dosierungsform handelt, die 10 Gew.-% bis 33 Gew.-% Cefepim oder eines pharmazeutisch unbedenklichen Salzes davon umfasst oder
wobei es sich bei der pharmazeutischen Zusammensetzung um eine Tablette handelt, die ein Sprengmittel und eine hydrophile Matrix, gegebenenfalls ausgewählt aus Maltose, Mannitol, Saccharose, kristalliner Cellulose, Maisstärke und Methylcellulose, umfasst.

9. Pharmazeutische Zusammensetzung nach einem der Ansprüche 1-5, wobei die Einheitsdosisform 0,1 Gew.-% bis 60 Gew.-% Wasser, genauer 0,1 Gew.-% bis 15 Gew.-% Wasser, umfasst, wobei gegebenenfalls:
die Einheitsdosisform als Suspension zur Rekonstitution formuliert ist oder
die Einheitsdosisform als flüssigkeitsgefüllte Gelkapsel formuliert ist.

10. Pharmazeutische Zusammensetzung nach einem der Ansprüche 1-5, wobei das Cefepim oder ein pharmazeutisch unbedenkliches Salz davon und der Zusatz auf einer festen Matrix adsorbiert sind.

11. Pharmazeutische Zusammensetzung nach Anspruch 10, wobei es sich bei der festen Matrix um ein poröses Silikat handelt, wobei gegebenenfalls:
das poröse Silikat Aluminiumsilikat, Magnesiumaluminiumsilikat, Natriumsilikat, Kaliumsilikat, Magnesiumsilikat oder Calciumsilikat umfasst oder
die pharmazeutische Zusammensetzung 0,2 Gew.-% bis 5 Gew.-% Chloridsalz umfasst.

12. Pharmazeutische Zusammensetzung nach Anspruch 1, wobei es sich bei der pharmazeutischen Zusammensetzung um eine rekonstituierbare Mischung handelt, die 50 Gew.-% bis 75 Gew.-% Acylcarnitin und 15 Gew.-% bis 25 Gew.-% Cefepim oder ein Salz davon umfasst.

13. Pharmazeutische Zusammensetzung nach Anspruch 12:
wobei die pharmazeutische Zusammensetzung weiterhin 10 Gew.-% bis 20 Gew.-% Carbonsäure oder ein Salz davon umfasst und/oder
wobei es sich bei der pharmazeutischen Zusammensetzung um ein Pulver handelt.

14. Pharmazeutische Zusammensetzung nach einem der Ansprüche 1-13 zur Verwendung in einem Verfahren zur Behandlung einer bakteriellen Infektion in einem Subjekt, wobei das Verfahren die orale Verabreichung einer pharmazeutischen Zusammensetzung nach einem der Ansprüche 1 bis 13 an das Subjekt umfasst, wobei die Zusammensetzung in einer zur Behandlung der Infektion wirksamen Menge verabreicht wird.

## Revendications

1. Composition pharmaceutique dans une forme pharmaceutique orale comprenant du céfépime, ou un sel pharmaceutiquement acceptable de celui-ci, et un additif comprenant une acyl-carnitine ou un sel de celle-ci, dans laquelle ledit additif est présent en une quantité suffisante pour augmenter la biodisponibilité orale dudit céfépime, ou dudit sel pharmaceutiquement acceptable de celui-ci,
dans laquelle ladite forme pharmaceutique orale comprend un rapport (m/m) du céfépime, ou un sel pharmaceutiquement acceptable de celui-ci, à l'acyl-carnitine de 1:0,5 à 1:6.

2. Composition pharmaceutique selon la revendication 1, dans laquelle ladite forme pharmaceutique orale comprend un rapport (m/m) du céfépime, ou un sel pharmaceutiquement acceptable de celui-ci, à l'acyl-carnitine de 1:2 à 1:5.

3. Composition pharmaceutique selon l'une quelconque des revendications 1 ou 2, dans laquelle ledit additif comprend en outre un alcool, un tensioactif polysorbate, un acide carboxylique, un polyéthylène glycol, un glycéride polyglycolisé, des alkylsaccharides, des esters de saccharide, un composé TPGS, ou un sucre.

4. Composition pharmaceutique selon la revendication 3 :
dans laquelle ladite forme pharmaceutique unitaire comprend de 1 % à 60 % (m/m) d'un alcool choisi parmi le propylène glycol, l'éthanol ou le glycérol ;
dans laquelle ladite forme pharmaceutique unitaire comprend de 1 % à 10 % (m/m) d'un acide carboxylique choisi parmi l'acide citrique, l'acide succinique, l'acide tartrique, l'acide fumarique, l'acide maléique, l'acide malonique, l'acide glutarique, l'acide adipique, l'acide lactique, l'acide malique, l'acide L-glutamique, l'acide L-aspartique, l'acide gluconique, l'acide glucuronique, l'acide salicylique, et des sels de ceux-ci ;
dans laquelle ladite forme pharmaceutique unitaire comprend de 0,5 % à 10 % (m/m) d'un tensioactif polysorbate ;
dans laquelle ladite forme pharmaceutique unitaire comprend de 0,2 % à 12 % (m/m) de composé TPGS ;
dans laquelle ladite forme pharmaceutique unitaire comprend de 2 % à 30 % (m/m) de polyéthylène glycol ;
dans laquelle ladite forme pharmaceutique unitaire comprend de 5 % à 30 % (m/m) d'alkylsaccharide ou d'ester de saccharide ;
dans laquelle ladite forme pharmaceutique unitaire comprend de 5 % à 60 % (m/m) de glycéride polyglycolisé ; ou
dans laquelle ladite forme pharmaceutique unitaire comprend de 5 % à 25 % (m/m) de sucre choisi parmi des monosaccharides, des disaccharides, et des alcools de sucre.

5. Composition pharmaceutique selon l'une quelconque des revendications 1 à 4, dans laquelle ladite acyl-carnitine est choisie parmi l'oléoyl-carnitine, la palmitoyl-carnitine, la décanoyl-carnitine, la dodécanoyl-carnitine, la myristoyl-carnitine, la stéaroyl-carnitine, et des sels de celles-ci.

6. Composition pharmaceutique selon l'une quelconque des revendications 1 à 5 :
dans laquelle ladite forme pharmaceutique unitaire est formulée sous la forme d'une capsule de gel remplie de liquide ou sous la forme d'un sachet pour reconstitution ; ou
dans laquelle ladite forme pharmaceutique unitaire comprend un solide ou un semi-solide, tel qu'une poudre.

7. Composition pharmaceutique selon l'une quelconque des revendications 1 à 6, ladite forme pharmaceutique orale comprenant de 5 % à 50 % (m/m) d'acyl-carnitine ou un sel pharmaceutiquement acceptable de celle-ci.

8. Composition pharmaceutique selon la revendication 7 ;
ladite composition pharmaceutique étant une forme pharmaceutique liquide comprenant de 1,5 % à 15 % (m/m) de céfépime ou un sel pharmaceutiquement acceptable de celle-ci ;
ladite composition pharmaceutique étant une forme pharmaceutique solide ou semi-solide comprenant de 10 % à 33 % (m/m) de céfépime ou un sel pharmaceutiquement acceptable de celui-ci ; ou
ladite composition pharmaceutique étant un comprimé comprenant un délitant et une matrice hydrophile, facultativement choisie parmi le maltose, le mannitol, le saccharose, la cellulose cristalline, l'amidon de maïs et la méthylcellulose.

9. Composition pharmaceutique selon l'une quelconque des revendications 1 à 5, dans laquelle ladite forme pharmaceutique unitaire comprend de 0,1 % à 60 % (m/m) d'eau, plus spécifiquement de 0,1 % (m/m) à 15 % (m/m) d'eau, facultativement :
dans laquelle ladite forme pharmaceutique unitaire est formulée sous la forme d'une suspension pour reconstitution ; ou
dans laquelle ladite forme pharmaceutique unitaire est formulée sous la forme d'une capsule de gel remplie de liquide.

10. Composition pharmaceutique selon l'une quelconque des revendications 1 à 5, dans laquelle ledit céfépime, ou un sel pharmaceutiquement acceptable de celui-ci, et ledit additif sont adsorbés sur une matrice solide.

11. Composition pharmaceutique selon la revendication 10, dans laquelle ladite matrice solide est un silicate poreux, facultativement :
dans laquelle ledit silicate poreux comprend le silicate d'aluminium, le silicate de magnésium-aluminium, le silicate de sodium, le silicate de potassium, le silicate de magnésium ou le silicate de calcium ; ou
ladite composition pharmaceutique comprenant de 0,2 % à 5 % (m/m) de sel de chlorure.

12. Composition pharmaceutique selon la revendication 1, ladite composition pharmaceutique étant un mélange reconstituable comprenant de 50 % (m/m) à 75 % (m/m) d'acyl-carnitine et de 15 % (m/m) à 25 % (m/m) de céfépime, ou un sel de celui-ci.

13. Composition pharmaceutique selon la revendication 12 :
dans laquelle ladite composition pharmaceutique comprend en outre de 10 % (m/m) à 20 % (m/m) d'acide carboxylique ou un sel de celui-ci ; et/ou
ladite composition pharmaceutique étant une poudre.

14. Composition pharmaceutique selon l'une quelconque des revendications 1 à 13 pour utilisation dans un procédé de traitement d'une infection bactérienne chez un sujet, ledit procédé comprenant l'administration par voie orale audit sujet d'une composition pharmaceutique selon l'une quelconque des revendications 1 à 13, ladite composition étant administrée en une quantité efficace pour traiter ladite infection.
